(19) Europäisches Patentamt
European Patent Office
Office européen des brevets

(11) EP 4 059 503 A1

(12) # EUROPEAN PATENT APPLICATION
published in accordance with Art. 153(4) EPC

(43) Date of publication:
**21.09.2022 Bulletin 2022/38**

(21) Application number: **20888347.0**

(22) Date of filing: **12.11.2020**

(51) International Patent Classification (IPC):
***A61K 31/496*** *(2006.01)* ***A61K 31/4545*** *(2006.01)*
***A61K 31/497*** *(2006.01)* ***A61P 1/02*** *(2006.01)*
***A61P 1/04*** *(2006.01)* ***A61P 1/10*** *(2006.01)*

(52) Cooperative Patent Classification (CPC):
**A61K 31/4545; A61K 31/496; A61K 31/497; A61P 1/02; A61P 1/04; A61P 1/10**

(86) International application number:
**PCT/JP2020/042259**

(87) International publication number:
**WO 2021/095805 (20.05.2021 Gazette 2021/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2019 JP 2019205436**

(71) Applicant: **Nippon Shinyaku Co., Ltd.**
**Kyoto-shi**
**Kyoto 601-8550 (JP)**

(72) Inventors:
- **YOSHINAGA, Ryohei**
**Kyoto-shi, Kyoto 601-8550 (JP)**
- **KURITA, Maki**
**Kyoto-shi, Kyoto 601-8550 (JP)**
- **ASAO, Yasunori**
**Kyoto-shi, Kyoto 601-8550 (JP)**
- **FUKUI, Tomomi**
**Kyoto-shi, Kyoto 601-8550 (JP)**

(74) Representative: **Vossius & Partner**
**Patentanwälte Rechtsanwälte mbB**
**Siebertstraße 3**
**81675 München (DE)**

(54) **THERAPEUTIC AGENT AND PROPHYLACTIC AGENT FOR FUNCTIONAL GASTROINTESTINAL DISORDERS AND XEROSTOMIA**

(57) A novel prophylactic agent or therapeutic agent for functional gastrointestinal disorders or xerostomia is provided. The present invention is a therapeutic agent or prophylactic agent for functional gastrointestinal disorders, containing an azabenzimidazole compound represented by the following formula [1] (each symbol in the formula is as described in the specification), or a pharmaceutically acceptable salt thereof, or a solvate thereof, as an active ingredient.

[Chem. 1]

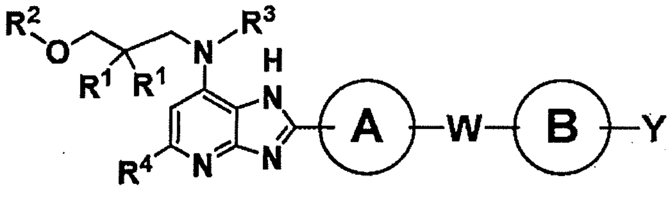

**[ 1 ]**

EP 4 059 503 A1

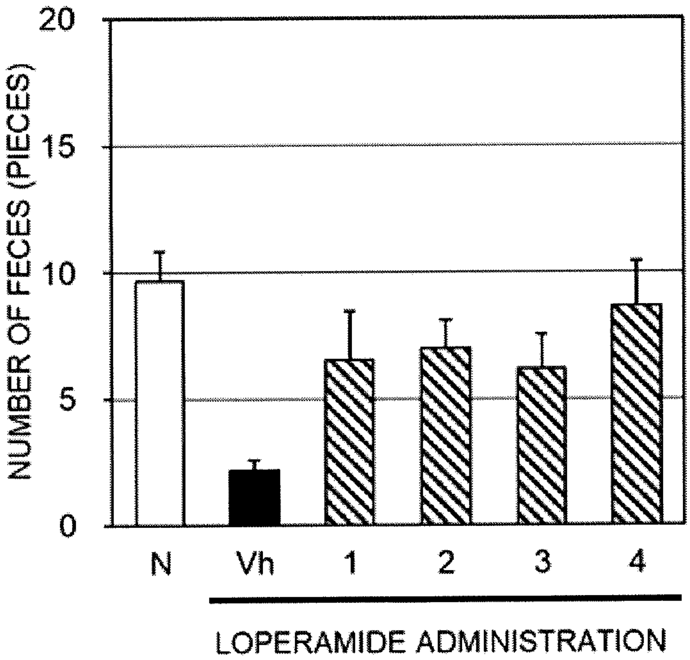
20
15
10
5
0
NUMBER OF FECES (PIECES)
N
Vh
1
2
3
4
LOPERAMIDE ADMINISTRATION

Fig.1

## Description

TECHNICAL FIELD

The present invention relates to a therapeutic agent and a prophylactic agent for functional gastrointestinal disorders (FGIDs). The present invention also relates to a therapeutic agent and a prophylactic agent for xerostomia (dry mouth).

According to Rome IV diagnostic criteria (hereinafter, sometimes referred to as "Rome IV"), functional gastrointestinal disorders are also called disorders of gut - brain interaction, and are considered to be a group of disorders that manifest gastrointestinal symptoms related to gastrointestinal motility abnormalities, visceral hyperesthesia, etc. (NON-PATENT DOCUMENT 1).

Adult functional gastrointestinal disorders include esophageal disorders, gastroduodenal disorders, bowel disorders, centrally mediated disorders of gastrointestinal pain gallbladder and sphincter of Oddi disorders, and anorectal disorders (NON-PATENT DOCUMENTS 1 and 2). For example, esophageal disorders include functional heartburn, gastroduodenal disorders include functional dyspepsia, and bowel disorders include irritable bowel syndrome (IBS), functional constipation, and opioid-induced constipation.

The onset and pathological conditions of functional gastrointestinal disorders are considered to be closely related to physiological functions such as gastrointestinal motility, sensation, and microflora, psychological factors such as stress, and genetics, and environmental factors, etc. (NON-PATENT DOCUMENT 1). Patients with functional gastrointestinal disorders have chronic or recurrent gastrointestinal symptoms (for example, upset stomach, stomach pain, diarrhea or constipation, etc.), and such patients have decreased quality of life (QOL) due to restricted daily activities.

Xerostomia is dryness of oral cavity result from the insufficient saliva secretion due to various causes. When the saliva secretion decreases, objective symptoms such as bad breath are observed in addition to unpleasant subjective symptoms such as masticatory disorders, dysgeusia, and dry mouse, and tongue coating and periodontal disease are observed. When the decrease in the amount of saliva secreted is severe, decayed teeth, angular cheilitis, etc., are observed in the patients. Therefore, the QOL of patients with xerostomia decreases (NON-PATENT DOCUMENT 3).

PRIOR ART DOCUMENTS

[NON-PATENT DOCUMENTS]

NON-PATENT DOCUMENT 1: Rome IV (Functional Gastrointestinal Disorders, 4th Edition), 2016, Rome Foundation, INC.

NON-PATENT DOCUMENT 2: Gastroenterology, 2016, Vol. 150, No. 6, p. 1257-1261

NON-PATENT DOCUMENT 3: Advances in Clinical Experimental Medicine, 2016, Vol. 25, No. 1, p. 199-206

NON-PATENT DOCUMENT 4: Pharmacolical Reviews, 1998, Vol. 50, No. 2, p. 279-290

NON-PATENT DOCUMENT 5: British Journal of Pharmacology, 2006, Vol. 148, No. 5, p. 565-578

NON-PATENT DOCUMENT 6: Trends in Pharmacological Sciences, 2017, Vol. 38, No. 9, p. 837-847

NON-PATENT DOCUMENT 7: Nature, 2012, Vol. 482, p. 552-556


SUMMARY OF THE INVENTION

PROBLEMS TO BE SOLVED BY THE INVENTION

As described above, functional gastrointestinal disorders and xerostomia decrease the patient’s quality of life. Therefore, therapeutic agents and prophylactic agents for functional gastrointestinal disorders and xerostomia are desired.

An object of the present invention is to provide a novel therapeutic agent or prophylactic agent for functional gastrointestinal disorders. Another object of the present invention is to provide a novel therapeutic agent or prophylactic agent for xerostomia.

MEANS OF SOLVING THE PROBLEMS

As a result of intensive studies, the inventors discovered that an azabenzimidazole compound represented by the following formula [1], or a pharmaceutically acceptable salt thereof, or a solvate thereof (sometimes herein referred to as a "compound of the present invention") is useful for the prevention and treatment of functional gastrointestinal

disorders and xerostomia, and achieved the present invention.

That is, for the present invention, the following (Item 1) to (Item 14) can be mentioned.

(Item 1)

A therapeutic agent or prophylactic agent for a functional gastrointestinal disorder, comprising an azabenzimidazole compound, or a pharmaceutically acceptable salt thereof, or a solvate thereof, as an active ingredient, the azabenzimidazole compound being a compound of the formula [1]:

[Chem. 1]

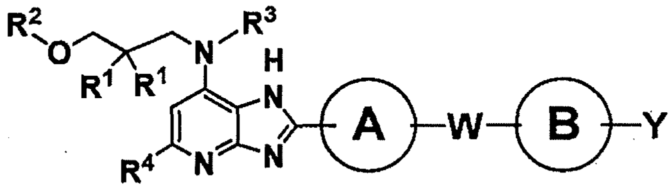

[1]

wherein:

$R^1$ is a hydrogen atom or alkyl, or the two $R^1$s combine with the adjacent carbon atom to form a 3- to 7-membered cycloalkyl or an oxygen-containing non-aromatic heterocycle;
$R^2$ is a hydrogen atom, alkyl, cycloalkyl, alkyl substituted with cycloalkyl, or alkoxyalkyl;
$R^3$ is a hydrogen atom, alkyl, or alkoxyalkyl;
$R^4$ is pyridyl optionally substituted with one or two groups selected from the group consisting of alkyl, trihaloalkyl, alkoxy, cyano, and cycloalkyl, or phenyl optionally substituted with 1 to 3 groups selected from the group consisting of trihaloalkyl, halogen, alkoxy, and cycloalkyl;
A is a group of the formula A-1, A-2, A-3, A-4, or A-5:

[Chem. 2]

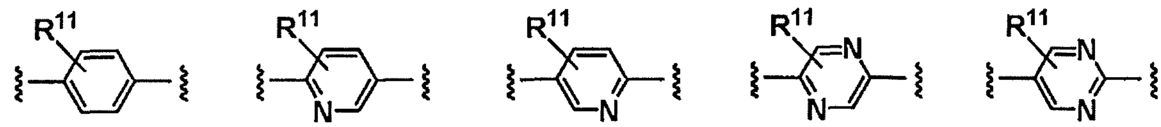

A-1 A-2 A-3 A-4 A-5

wherein the bond on the left side of each group is attached to the 2-position of the azabenzimidazole in the formula [1], the bond on the right side is attached to W in the formula [1], and $R^{11}$ is a group selected from a hydrogen atom, halogen, alkyl, alkoxy, and nitro; W is a bond or a group of the formula W-1, W-2, or W-3:

[Chem. 3]

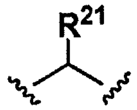

W-1

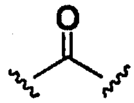

W-2

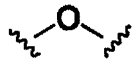

W-3

wherein $R^{21}$ is a hydrogen atom or alkyl;
B is a group of the formula B-1, B-2, B-3, or B-4:

[Chem. 4]

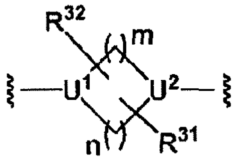

B-1

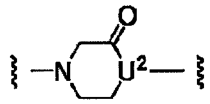

B-2

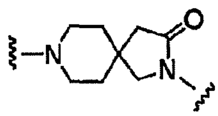

B-3

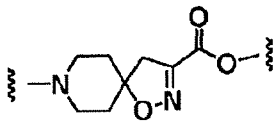

B-4

wherein the bond on the left side of each group is attached to W in the formula [1], the bond on the right side is attached to Y in the formula [1], $U^1$ is a nitrogen atom or $CR^{41}$, $U^2$ is a nitrogen atom or $CR^{42}$, $R^{41}$ and $R^{42}$ are each independently a hydrogen atom, alkyl, halogen, or a hydroxyl group, m and n are each 1, 2, or 3, and $R^{31}$ and $R^{32}$ are each independently a hydrogen atom, alkyl, halogen, or alkoxyalkyl, or $R^{31}$ and $R^{32}$ combine with an adjacent carbon atom to form an alkylene bridge, provided that $R^{31}$ and $R^{32}$ substitute at any substitutable positions other than $U^1$ and $U^2$; and
Y is a hydrogen atom or a group of any one of the formulae Y-1 to Y-4, Y-11 to Y-16:

[Chem. 5]

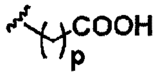

Y-1

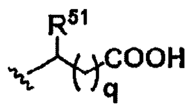

Y-2

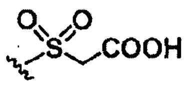

Y-3

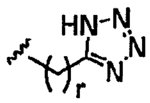

Y-4

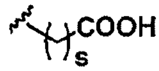

Y-11

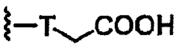

Y-12

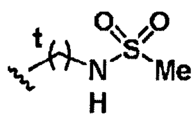

Y-13

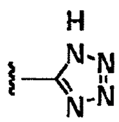

Y-14

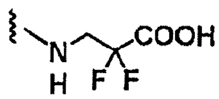

Y-15

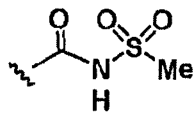

Y-16

wherein $R^{51}$ is alkyl, p is 1, 2, or 3, q is 0, 1, or 2, r is 1, 2, or 3, T is O, S, $SO_2$, or $NR^{61}$ wherein $R^{61}$ is a hydrogen atom or alkyl, s is 0, 1, 2, or 3, and t is 0 or 1, with the proviso that one of the following cases (a) to (d) is selected:

(a) when W is a bond,

if B is B-1 or B-2 and $U^2$ is a nitrogen atom, then Y is Y-1, Y-2, Y-3, or Y-4,

if B is B-1 or B-2 and $U^2$ is $CR^{42}$ wherein $R^{42}$ is as defined above, then $U^1$ is a nitrogen atom and Y is Y-11, Y-12, Y-13, Y-14, Y-15, or Y-16, and
if B is B-3 or B-4, then Y is a hydrogen atom;

(b) when W is W-1,

if B is B-1, $U^1$ is a nitrogen atom, and $U^2$ is a nitrogen atom, then Y is Y-1, Y-2, Y-3, or Y-4, and
if B is B-1, $U^1$ is a nitrogen atom, and $U^2$ is $CR^{42}$ wherein $R^{42}$ is as defined above, then Y is Y-11, Y-12, Y-13, Y-14, Y-15, or Y-16;

(c) when W is W-2,

if B is B-1 or B-2, $U^1$ is a nitrogen atom, and $U^2$ is a nitrogen atom, then Y is Y-1, Y-2, Y-3, or Y-4,
if B is B-1 or B-2, $U^1$ is a nitrogen atom, and $U^2$ is $CR^{42}$ wherein $R^{42}$ is as defined above, then Y is Y-11, Y-12, Y-13, Y-14, Y-15, or Y-16, and
if B is B-3 or B-4, then Y is a hydrogen atom; and

(d) when W is W-3,

if B is B-1, $U^1$ is $CR^{41}$ wherein $R^{41}$ is as defined above, and $U^2$ is a nitrogen atom, then Y is Y-1, Y-2, Y-3, or Y-4.

(Item 2)

The therapeutic agent or prophylactic agent according to Item 1, wherein W is a bond.

(Item 3)

The therapeutic agent or prophylactic agent according to Item 1 or 2, wherein

(1) B is B-1 or B-2, $U^2$ is a nitrogen atom, and Y is Y-1, Y-2, or Y-3,
(2) B is B-1 or B-2, $U^2$ is $CR^{42}$, and Y is Y-11, Y-12, or Y-15, or
(3) B is B-4 and Y is a hydrogen atom.

(Item 4)

The therapeutic agent or prophylactic agent according to Item 3, wherein $R^4$ is pyridyl substituted with trihaloalkyl and a group selected from the group consisting of alkyl, trihaloalkyl, alkoxy, cyano, and cycloalkyl.

(Item 5)

The therapeutic agent or prophylactic agent according to Item 4, wherein A is A-4.

(Item 6)

The therapeutic agent or prophylactic agent according to any one of Items 1 to 5, wherein the azabenzimidazole compound is any one of the following (1) to (15):

(1) [4-(5-{5-[3-fluoro-5-(trifluoromethyl)phenyl]-7-[{[1-(methoxymethyl)cyclobutyl]methyl}(methyl)amino]-1H-imidazo [4,5 -b]pyridin-2-yl} pyrazin-2-yl)piperazin-1-yl]acetic acid,
(2) 4-fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,
(3) 1-(5-{5-[6-ethoxy-5-(trifluoromethyl)pyridin-3-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,
(4) 1-(5-{5- [2-ethoxy-6-(trifluoromethyl)pyridin-4-yl] -7-[{[1-(methoxymethyl)cyclobutyl]methyl}(methyl)amino]-1H-imidazo[4,5-b]pyridin-2-yl}pyrazin-2-yl)piperidine-4-carboxylic acid,
(5) {[1-(5-{5-[2-cyclopropyl-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidin-4-yl]oxy}acetic acid,
(6) 1 -(4- {5 - [6-ethoxy-5 -(trifluoromethyl)pyridin-3-yl]-7-[{[1-(methoxymethyl)cyclopentyl]methyl} (methyl)amino]-

1H-imidazo[4,5-b]pyridin-2-yl}-3-fluorophenyl)piperidine-4-carboxylic acid,
(7) 1-(5-{5-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylic acid,
(8) 1-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)piperidine-4-carboxylic acid,
(9) 1-(5-{5-[2-ethoxy-6-(trifluoromethyl)pyridin-4-yl]-7-[{[1-(methoxymethyl)cyclopentyl]methyl} (methyl)amino]-1H-imidazo[4,5-b]pyridin-2-yl} pyrazin-2-yl)piperidine-4-carboxylic acid,
(10) 3-[4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl]propanoic acid,
(11) [4-(4-{5-[6-cyclopropyl-5-(trifluoromethyl)pyridin-3-yl]-7-[{[1-(methoxymethyl)cyclopentyl]methyl}(methyl)amino]-1H-imidazo [4,5-b]pyridin-2-yl}-3-fluorophenoxy)piperidin-1-yl]acetic acid,
(12) 3-[(2S)-4-(5-{5-[6-cyclopropyl-5-(trifluoromethyl)pyridin-3-yl]-7-[([1-(methoxymethyl)cyclohexyl]methyl} (methyl)amino]-1H-imidazo[4,5-b]pyridin-2-yl}pyrazin-2-yl)-2-(methoxymethyl)piperazin-1-yl]propanoic acid,
(13) 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoic acid,
(14) 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid, and
(15) 3-[(3R)-4-(5-{5-[6-cyclopropyl-5-(trifluoromethyl)pyridin-3-yl]-7-[{[1-(methoxymethyl)cyclopentyl]methyl} (methyl)amino]-1H-imidazo[4,5-b]pyridin-2-yl} pyrazin-2-yl)-3-methylpiperazin-1-yl]propanoic acid.

(Item 7)

The therapeutic agent or prophylactic agent according to any one of Items 1 to 6, wherein the functional gastrointestinal disorder is irritable bowel syndrome (IBS).

(Item 8)

The therapeutic agent or prophylactic agent according to any one of Items 1 to 6, wherein the functional gastrointestinal disorder is functional constipation.

(Item 9)

A therapeutic agent or prophylactic agent for xerostomia, comprising an azabenzimidazole compound, or a pharmaceutically acceptable salt thereof, or a solvate thereof, as an active ingredient, the azabenzimidazole compound being a compound of the formula [1]:

[Chem. 6]

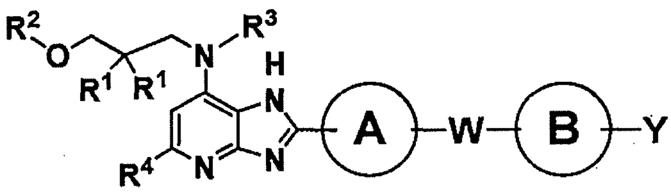

[1]

wherein:

$R^1$ is a hydrogen atom or alkyl, or the two $R^1$s combine with the adjacent carbon atom to form a 3- to 7-membered cycloalkyl or an oxygen-containing non-aromatic heterocycle;
$R^2$ is a hydrogen atom, alkyl, cycloalkyl, alkyl substituted with cycloalkyl, or alkoxyalkyl;
$R^3$ is a hydrogen atom, alkyl, or alkoxyalkyl;
$R^4$ is pyridyl optionally substituted with one or two groups selected from the group consisting of alkyl, trihaloalkyl, alkoxy, cyano, and cycloalkyl, or phenyl optionally substituted with 1 to 3 groups selected from the group consisting

of trihaloalkyl, halogen, alkoxy, and cycloalkyl;
A is a group of the formula A-1, A-2, A-3, A-4, or A-5:

[Chem. 7]

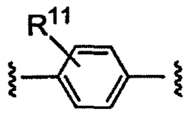
A-1

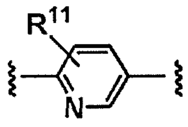
A-2

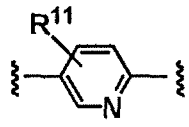
A-3

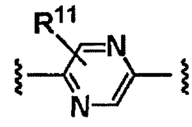
A-4

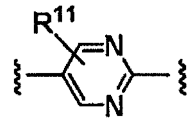
A-5

wherein the bond on the left side of each group is attached to the 2-position of the azabenzimidazole in the formula [1], the bond on the right side is attached to W in the formula [1], and $R^{11}$ is a group selected from a hydrogen atom, halogen, alkyl, alkoxy, and nitro; W is a bond or a group of the formula W-1, W-2, or W-3:

[Chem. 8]

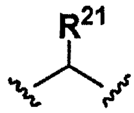
W-1

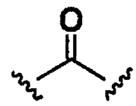
W-2

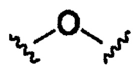
W-3

wherein $R^{21}$ is a hydrogen atom or alkyl;
B is a group of the formula B-1, B-2, B-3, or B-4:

[Chem. 9]

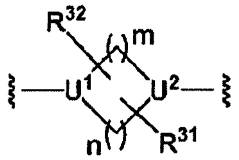
B-1

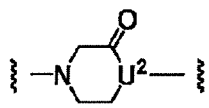
B-2

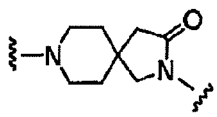
B-3

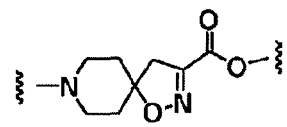
B-4

wherein the bond on the left side of each group is attached to W in the formula [1], the bond on the right side is attached to Y in the formula [1], $U^1$ is a nitrogen atom or $CR^{41}$, $U^2$ is a nitrogen atom or $CR^{42}$, $R^{41}$ and $R^{42}$ are each independently a hydrogen atom, alkyl, halogen, or a hydroxyl group, m and n are each 1, 2, or 3, and $R^{31}$ and $R^{32}$ are each independently a hydrogen atom, alkyl, halogen, or alkoxyalkyl, or $R^{31}$ and $R^{32}$ combine with an adjacent carbon atom to form an alkylene bridge, provided that $R^{31}$ and $R^{32}$ substitute at any substitutable positions other than $U^1$ and $U^2$; and
Y is a hydrogen atom or a group of any one of the formulae Y-1 to Y-4, Y-11 to Y-16:

[Chem. 10]

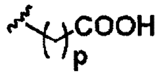
**Y-1**

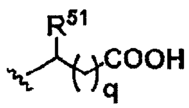
**Y-2**

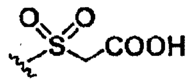
**Y-3**

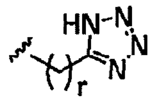
**Y-4**

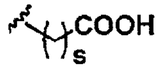
**Y-11**

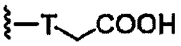
**Y-12**

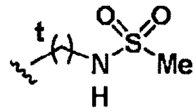
**Y-13**

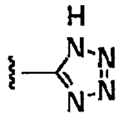
**Y-14**

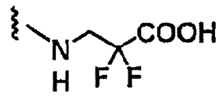
**Y-15**

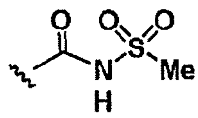
**Y-16**

wherein $R^{51}$ is alkyl, p is 1, 2, or 3, q is 0, 1, or 2, r is 1, 2, or 3, T is O, S, $SO_2$, or $NR^{61}$ wherein $R^{61}$ is a hydrogen atom or alkyl, s is 0, 1, 2, or 3, and t is 0 or 1, with the proviso that one of the following cases (a) to (d) is selected:

(a) when W is a bond,

if B is B-1 or B-2 and $U^2$ is a nitrogen atom, then Y is Y-1, Y-2, Y-3, or Y-4,
if B is B-1 or B-2 and $U^2$ is $CR^{42}$ wherein $R^{42}$ is as defined above, then $U^1$ is a nitrogen atom and Y is Y-11, Y-12, Y-13, Y-14, Y-15, or Y-16, and
if B is B-3 or B-4, then Y is a hydrogen atom;

(b) when W is W-1,

if B is B-1, $U^1$ is a nitrogen atom, and $U^2$ is a nitrogen atom, then Y is Y-1, Y-2, Y-3, or Y-4, and
if B is B-1, $U^1$ is a nitrogen atom, and $U^2$ is $CR^{42}$ wherein $R^{42}$ is as defined above, then Y is Y-11, Y-12, Y-13, Y-14, Y-15, or Y-16;

(c) when W is W-2,

if B is B-1 or B-2, $U^1$ is a nitrogen atom, and $U^2$ is a nitrogen atom, then Y is Y-1, Y-2, Y-3, or Y-4,
if B is B-1 or B-2, $U^1$ is a nitrogen atom, and $U^2$ is $CR^{42}$ wherein $R^{42}$ is as defined above, then Y is Y-11, Y-12, Y-13, Y-14, Y-15, or Y-16, and
if B is B-3 or B-4, then Y is a hydrogen atom; and

(d) when W is W-3,

if B is B-1, $U^1$ is $CR^{41}$ wherein $R^{41}$ is as defined above, and $U^2$ is a nitrogen atom, then Y is Y-1, Y-2, Y-3, or Y-4.

(Item 10)

The therapeutic agent or prophylactic agent according to Item 9, wherein W is a bond.

(Item 11)

The therapeutic agent or prophylactic agent according to Item 9 or 10, wherein

(1) B is B-1 or B-2, $U^2$ is a nitrogen atom, and Y is Y-1, Y-2, or Y-3,
(2) B is B-1 or B-2, $U^2$ is $CR^{42}$, and Y is Y-11, Y-12, or Y-15, or
(3) B is B-4 and Y is a hydrogen atom.

(Item 12)

The therapeutic agent or prophylactic agent according to Item 11, wherein $R^4$ is pyridyl substituted with trihaloalkyl and a group selected from the group consisting of alkyl, trihaloalkyl, alkoxy, cyano, and cycloalkyl.

(Item 13)

The therapeutic agent or prophylactic agent according to Item 12, wherein A is A-4.

(Item 14)

The therapeutic agent or prophylactic agent according to any one of Items 9 to 13, wherein the azabenzimidazole compound is any one of the following (1) to (15):

(1) [4-(5-{5-[3-fluoro-5-(trifluoromethyl)phenyl]-7-[{[1-(methoxymethyl)cyclobutyl]methyl} (methyl)amino]-1H-imidazo[4,5-b]pyridin-2-yl} pyrazin-2-yl)piperazin-1-yl]acetic acid,
(2) 4-fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,
(3) 1-(5-{5-[6-ethoxy-5-(trifluoromethyl)pyridin-3-yl]-7-[{[1-(methoxymethyl)cyclobutyl]methyl} (methyl)amino]-1H-imidazo[4,5-b]pyridin-2-yl} pyrazin-2-yl)piperidine-4-carboxylic acid,
(4) 1-(5-{5 - [2-ethoxy-6-(trifluoromethyl)pyridin-4-yl] -7-[{[1-(methoxymethyl)cyclobutyl]methyl} (methyl)amino]-1H-imidazo[4,5-b]pyridin-2-yl}pyrazin-2-yl)piperidine-4-carboxylic acid,
(5) {[1-(5-{5-[2-cyclopropyl-6-(trifluoromethyl)pyridin-4-yl]-7-[{[1-(methoxymethyl)cyclopentyl]methyl} (methyl)amino] -1H-imidazo [4,5-b]pyridin-2-yl} pyrazin-2-yl)piperidin-4-yl]oxy}acetic acid,
(6) 1 -(4- {5 - [6-ethoxy-5-(trifluoromethyl)pyridin-3 -yl]-7-[{[1-(methoxymethyl)cyclopentyl]methyl} (methyl)amino] -1H-imidazo [4,5-b]pyridin-2-yl} -3-fluorophenyl)piperidine-4-carboxylic acid,
(7) 1-(5-{5-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylic acid,
(8) 1-(5-{5-[6-cyclopropyl-5-(trifluoromethyl)pyridin-3-yl]-7-[{[1-(ethoxymethyl)cyclopentyl]methyl} (methyl)amino]-1H-imidazo[4,5-b]pyridin-2-yl}pyrazin-2-yl)piperidine-4-carboxylic acid,
(9) 1-(5-{5-[2-ethoxy-6-(trifluoromethyl)pyridin-4-yl]-7-[{[1-(methoxymethyl)cyclopentyl]methyl} (methyl)amino]-1H-imidazo[4,5-b]pyridin-2-yl}pyrazin-2-yl)piperidine-4-carboxylic acid,
(10) 3-[4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl]propanoic acid,
(11) [4-(4-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}-3-fluorophenoxy)piperidin-1-yl] acetic acid,
(12) 3-[(2S)-4-(5-{5-[6-cyclopropyl-5-(trifluoromethyl)pyridin-3-yl]-7-[{[1-(methoxymethyl)cyclohexyl]methyl} (methyl)amino]-1H-imidazo[4,5-b]pyridin-2-yl} pyrazin-2-yl)-2-(methoxymethyl)piperazin-1-yl]propanoic acid,
(13) 3-[(3R)-4-(5-{5-[6-cyclopropyl-5-(trifluoromethyl)pyridin-3-yl]-7-[{[1-(ethoxymethyl)cyclopentyl]methyl} (methyl)amino]-1H-imidazo[4,5-b]pyridin-2-yl}pyrazin-2-yl)-3-methylpiperazin-1-yl]propanoic acid,
(14) 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid, and
(15) 3-[(3R)-4-(5-15-[6-cyclopropyl-5-(trifluoromethyl)pyridin-3-yl]-7-[{[1-(methoxymethyl)cyclopentyl]methyl} (methyl)amino]-1H-imidazo [4,5-b]pyridin-2-yl}pyrazin-2-yl)-3-methylpiperazin-1-yl]propanoic acid.

ADVANTAGEOUS EFFECTS OF THE INVENTION

According to the present invention, it is possible to provide a novel therapeutic agent or prophylactic agent for functional gastrointestinal disorders or xerostomia, containing an azabenzimidazole compound represented by the formula [1], or a pharmaceutically acceptable salt thereof, or a solvate thereof, as an active ingredient.

BRIEF DESCRIPTION OF THE DRAWINGS

[FIG. 1] FIG. 1 shows the numbers of feces of constipation model mice to which compounds of Examples 1 to 4 were administered.
[FIG. 2] FIG. 2 shows the numbers of feces of constipation model mice to which compounds of Examples 6 and 7 were administered.
[FIG. 3] FIG. 3 shows the numbers of feces of constipation model mice to which compounds of Examples 8 to 11 were administered.
[FIG. 4] FIG. 4 shows the numbers of feces of constipation model mice to which compounds of Examples 12 to 15 were administered.

[FIG. 5] FIG. 5 shows the dry weight of feces in the cases of FIG. 1.
[FIG. 6] FIG. 6 shows the dry weight of feces in the cases of FIG. 2.
[FIG. 7] FIG. 7 shows the dry weight of feces in the cases of FIG. 3.
[FIG. 8] FIG. 8 shows the dry weight of feces in the cases of FIG. 4.

MODE FOR CARRYING OUT THE INVENTION

The meaning of each term as used herein is described below. Unless otherwise specified, each term is used in the same meaning when used alone or in combination with other terms.
"Halogen" refers to a fluorine atom, a chlorine atom, a bromine atom, and an iodine atom.
Examples of "alkyl" include linear or branched alkyl having 1 to 10 carbon atoms, preferably 1 to 8 carbon atoms, and more preferably 1 to 6 carbon atoms. Specific examples of "alkyl" include methyl, ethyl, n-propyl, isopropyl, n-butyl, isobutyl, sec-butyl, tert-butyl, n-pentyl, sec-pentyl, 1-ethylpropyl, 1,2-dimethylpropyl, tert-pentyl, 2-methylbutyl, isopentyl, neopentyl, n-hexyl, sec-hexyl, 1-ethylbutyl, isohexyl, neohexyl, 1,1-dimethylbutyl, texyl, 2-ethylbutyl, 1,2,2-trimethylpropyl, 2,2-dimethylbutyl, n-heptyl, isoheptyl, n-octyl, and isooctyl.
Examples of the alkyl moieties of "alkoxyalkyl" and "alkyl substituted with cycloalkyl" include the same "alkyl" as described above.
"Trihaloalkyl" refers to a group in which the above "alkyl" is substituted with three "halogens" described above. Specific examples of "trihaloalkyl" include trifluoromethyl, trichloromethyl, and trifluoroethyl.
"Alkoxy" refers to a group in which the above "alkyl" is bound to an oxygen atom. Examples of "alkoxy" include linear or branched alkoxy having 1 to 8 carbon atoms and preferably 1 to 6 carbon atoms. Specific examples of "alkoxy" include methoxy, ethoxy, n-propoxy, isopropoxy, n-butoxy, isobutoxy, sec-butoxy, tert-butoxy, n-pentyloxy, n-hexyloxy, n-heptyloxy, and n-octyloxy.
Examples of the alkoxy moiety of "alkoxyalkyl" include the same "alkoxy" as described above.
Examples of "alkylene" include an alkylene having a linear or branched divalent hydrocarbon group having 1 to 6 carbon atoms. Specific examples of "alkylene" include methylene, ethylene, and propylene.
Examples of "cycloalkyl" include mono-, di-, and tri-cyclic saturated hydrocarbon groups having 3 to 10 carbon atoms. Monocyclic cycloalkyl having 3 to 6 carbon atoms is preferable. Specific examples include cyclopropyl, cyclobutyl, cyclopentyl, cyclohexyl, cycloheptyl, bicyclo[2.1.0]pentyl, bicyclo[2.2.1]heptyl, and bicyclo[2.2.2]octyl.
Examples of the cycloalkyl moiety of "alkyl substituted with cycloalkyl" include the same "cycloalkyl" as described above.
Examples of "oxygen-containing non-aromatic heterocyclic group" include a 3- to 8-membered non-aromatic heterocyclic group, more preferably 5- to 7-membered non-aromatic heterocyclic group, containing an oxygen atom as a ring-constituting atom in addition to carbon atoms. Specific examples of "oxygen-containing non-aromatic heterocyclic group" include oxolanyl (1-oxolanyl, 2-oxolanyl), oxanyl (1-oxanyl, 2-oxanyl, 3-oxanyl), and oxepanyl (1-oxepanyl, 2-oxepanyl, 3-oxepanyl).
Hereinafter, each symbol in the formula [1] is described.
In the formula [1], optionally, each $R^1$ is a hydrogen atom or alkyl, or the two $R^1$s combine with the adjacent carbon atom to form a 3- to 7-membered cycloalkyl or an oxygen-containing non-aromatic heterocycle.
The "alkyl" for $R^1$ is preferably methyl, ethyl, n-propyl, and n-butyl, and more preferably methyl and ethyl.
The "cycloalkyl" for $R^1$ is preferably cyclopropyl, cyclobutyl, cyclopentyl, cyclohexyl, and cycloheptyl, and more preferably cyclobutyl, cyclopentyl, and cyclohexyl.
The "oxygen-containing non-aromatic heterocyclic group" for $R^1$ is preferably 1-oxanyl, 2-oxanyl, and 3-oxanyl, and more preferably 3-oxanyl.
In the formula [1], $R^2$ is a hydrogen atom, alkyl, cycloalkyl, alkyl substituted with cycloalkyl, or alkoxyalkyl.
The "alkyl" for $R^2$ is preferably methyl, ethyl, n-propyl, n-butyl, and n-pentyl, and more preferably methyl, ethyl, n-propyl, and n-butyl.
The "cycloalkyl" for $R^2$ is preferably cyclopropyl and cyclobutyl.
The cycloalkyl of "alkyl substituted with cycloalkyl" for $R^2$ is preferably cyclobutyl and cyclopentyl, and more preferably cyclobutyl.
The alkyl of "alkyl substituted with cycloalkyl" for $R^2$ is preferably methyl and ethyl, and more preferably methyl.
The alkoxy of "alkoxyalkyl" for $R^2$ is preferably methoxy, ethoxy, n-propoxy, and isopropoxy, and more preferably methoxy and ethoxy.
The alkyl of "alkoxyalkyl" for $R^2$ is preferably methyl, ethyl, and propyl, and more preferably methyl and ethyl.
In the formula [1], $R^3$ is a hydrogen atom, alkyl, cycloalkyl, alkyl substituted with cycloalkyl, or alkoxyalkyl.
The "alkyl" for $R^3$ is preferably methyl, ethyl, and n-propyl, and more preferably methyl and ethyl.
The alkoxy of "alkoxyalkyl" for $R^3$ is preferably methoxy and ethoxy, and more preferably methoxy.
In the formula [1], $R^4$ is pyridyl optionally substituted with one or two groups selected from the group consisting

of alkyl, trihaloalkyl, alkoxy, cyano, and cycloalkyl, or phenyl optionally substituted with 1 to 3 groups selected from the group consisting of trihaloalkyl, halogen, alkoxy, and cycloalkyl.

The "alkyl" in pyridyl optionally substituted with one or two alkyls for $R^4$ is preferably methyl, ethyl, and n-propyl.

The "trihaloalkyl" in pyridyl optionally substituted with one or two alkyls for $R^4$ is preferably trifluoromethyl.

The "alkoxy" in pyridyl optionally substituted with one or two alkoxys for $R^4$ is preferably methoxy, ethoxy, n-propoxy, and n-butoxy, and more preferably ethoxy.

The "cycloalkyl" in pyridyl optionally substituted with one or two cycloalkyls for $R^4$ is preferably cyclopropyl and cyclobutyl, and more preferably cyclopropyl.

The "trihaloalkyl" in phenyl optionally substituted with 1 to 3 trihaloalkyls for $R^4$ is preferably trifluoromethyl.

The "halogen" in phenyl optionally substituted with 1 to 3 halogens for $R^4$ is preferably a chlorine atom, a bromine atom, and a fluorine atom, and more preferably a fluorine atom.

The "alkoxy" in phenyl optionally substituted with 1 to 3 alkoxys for $R^4$ is preferably methoxy, ethoxy, n-propoxy, isopropoxy, and n-butoxy, and more preferably methoxy and ethoxy.

The "cycloalkyl" with which phenyl is optionally substituted for $R^4$ is preferably cyclopropyl and cyclobutyl, and more preferably cyclopropyl.

$R^4$ is preferably pyridyl substituted with trihaloalkyl and one group selected from the group consisting of alkyl, trihaloalkyl, alkoxy, cyano, and cycloalkyl as described above.

In the formula [1], A is a group of the formula A-1, A-2, A-3, A-4, or A-5.

[Chem. 11]

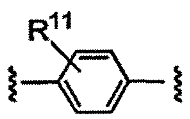
A-1

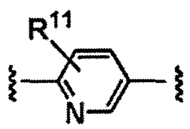
A-2

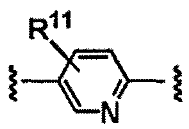
A-3

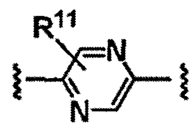
A-4

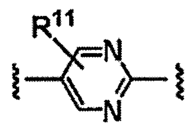
A-5

In the formula [1], $R^{11}$ is a group selected from a hydrogen atom, halogen, alkyl, alkoxy, and nitro.

The "halogen" for $R^{11}$ is preferably a chlorine atom, a bromine atom, and a fluorine atom, and more preferably a chlorine atom and a fluorine atom.

The "alkyl" for $R^{11}$ is preferably methyl, ethyl, and n-propyl, and more preferably methyl and ethyl.

The "alkoxy" for $R^{11}$ is preferably methoxy and ethoxy, and more preferably methoxy.

In the formula [1], A is preferably A-4.

In the formula [1], W is a bond, or selected from W-1, W-2, and W-3.

[Chem. 12]

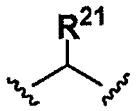
W-1

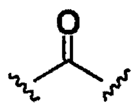
W-2

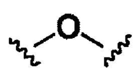
W-3

$R^{21}$ in W-1 is a group selected from a hydrogen atom and alkyl.

The "alkyl" for $R^{21}$ is preferably methyl and ethyl, and more preferably methyl.

W in the formula [1] is preferably a bond.

B is selected from B-1, B-2, B-3, and B-4.

[Chem. 13]

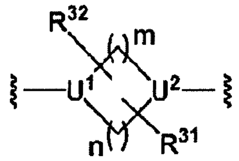 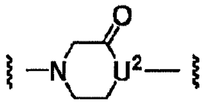 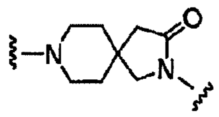 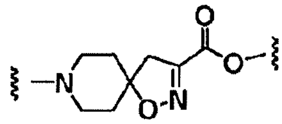

B-1 B-2 B-3 B-4

wherein the bond on the left side of each of B-1 to B-4 is attached to W in the formula [1], and the bond on the right side is attached to Y in the formula [1].

$U^1$ represents a nitrogen atom or $CR^{41}$, and $U^2$ represents a nitrogen atom or $CR^{42}$.

$R^{41}$ and $R^{42}$ each independently represent a hydrogen atom, alkyl, halogen, or a hydroxyl group.

m and n are each 1, 2 or 3.

$R^{31}$ and $R^{32}$ are each independently a hydrogen atom, alkyl, halogen, or alkoxyalkyl, or $R^{31}$ and $R^{32}$ may combine with an adjacent carbon atom to form an alkylene bridge.

$R^{31}$ and $R^{32}$ substitute at any substitutable positions other than $U^1$ and $U^2$.

The "alkyl" for $R^{31}$ and $R^{32}$ is preferably methyl and ethyl, and more preferably methyl.

The "halogen" for $R^{31}$ and $R^{32}$ is preferably a fluorine atom.

The "alkyl" of the "alkoxyalkyl" for $R^{31}$ and $R^{32}$ is preferably methyl, ethyl, and n-propyl, and more preferably methyl and ethyl.

The alkoxy of the "alkoxyalkyl" for $R^{31}$ and $R^{32}$ is preferably methoxy and ethoxy, and more preferably methoxy.

The alkylene bridge formed by $R^{31}$ and $R^{32}$ is preferably a linear alkylene bridge having 1 to 3 carbon atoms, and more preferably a methylene bridge or an ethylene bridge.

In the formula [1], B is preferably B-1, B-2, and B-4, more preferably B-1 and B-4, and further preferably B-1.

Y is a hydrogen atom, or selected from Y-1 to Y-4 and Y-11 to Y-16.

[Chem. 14]

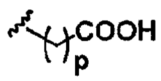 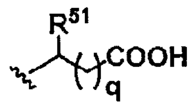 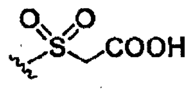 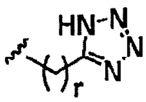 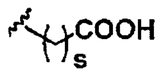

Y-1 Y-2 Y-3 Y-4 Y-11

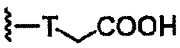 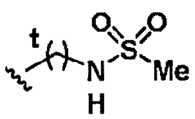 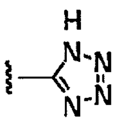 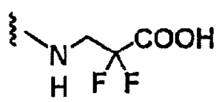 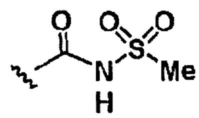

Y-12 Y-13 Y-14 Y-15 Y-16

$R^{51}$ is alkyl; p is 1, 2, or 3; q is 0, 1, or 2; r is 1, 2, or 3; T is O, S, $SO_2$, or $NR^{61}$ wherein $R^{61}$ is a hydrogen atom or alkyl; s is 0, 1, 2, or 3; and t is 0 or 1.

The "alkyl" for $R^{51}$ and $R^{61}$ is preferably methyl, ethyl, and n-propyl, and more preferably methyl and ethyl.

In the formula [1], Y is preferably Y-1, Y-2, Y-3, Y-11, Y-12, and Y-15.

As for the combination of W, B, and Y in the formula [1]:

(a) when W is a bond,

if B is B-1 or B-2 and $U^2$ is a nitrogen atom, then Y is Y-1, Y-2, Y-3, or Y-4, and preferably Y-1, Y-2, or Y-3,

if B is B-1 or B-2 and $U^2$ is $CR^{42}$ wherein $R^{42}$ is as defined above, then $U^1$ is a nitrogen atom and Y is Y-11, Y-12, Y-13, Y-14, Y-15, or Y-16, and preferably Y-11, Y-12, or Y-15, and
if B is B-3 or B-4, then Y is a hydrogen atom;

(b) when W is W-1,

if B is B-1, $U^1$ is a nitrogen atom, and $U^2$ is a nitrogen atom, then Y is Y-1, Y-2, Y-3, or Y-4, and preferably Y-1, Y-2, or Y-3, and
if B is B-1, $U^1$ is a nitrogen atom, and $U^2$ is $CR^{42}$ wherein $R^{42}$ is as defined above, then Y is Y-11, Y-12, Y-13, Y-14, Y-15, or Y-16, and preferably Y-11, Y-12, or Y-15;

(c) when W is W-2,

if B is B-1 or B-2, $U^1$ is a nitrogen atom, and $U^2$ is a nitrogen atom, then Y is Y-1, Y-2, Y-3, or Y-4, and preferably Y-1, Y-2, or Y-3,
if B is B-1 or B-2, $U^1$ is a nitrogen atom, and $U^2$ is $CR^{42}$ wherein $R^{42}$ is as defined above, then Y is Y-11, Y-12, Y-13, Y-14, Y-15, or Y-16, and preferably Y-11, Y-12, or Y-15, and
if B is B-3 or B-4, then Y is a hydrogen atom; and

(d) when W is W-3,

B is B-1,
$U^1$ is $CR^{41}$ wherein $R^{41}$ is as defined above,
$U^2$ is a nitrogen atom, and
Y is Y-1, Y-2, Y-3, or Y-4, and preferably Y-1, Y-2, or Y-3.

The compound of the present invention can be prepared from a known compound or an easily synthesizable intermediate, for example, according to the following method, Examples described below, or a known method. In the preparation of the compound of the present invention, in the case where a starting material has a substituent that affects the reaction, the reaction is generally carried out after protecting the starting material with a suitable protective group in advance by a known method. The protective group can be removed by a known method after the reaction.

The azabenzimidazole compound according to the present invention may be used as it is for pharmaceuticals, and can also be used in the form of a pharmaceutically acceptable salt or solvate, or a solvate of the salt, according to a known method. Examples of pharmaceutically acceptable salts include salts with mineral acids such as hydrochloric acid, hydrobromic acid, sulfuric acid, and phosphoric acid, salts with organic acids such as acetic acid, malic acid, lactic acid, citric acid, tartaric acid, maleic acid, succinic acid, fumaric acid, p-toluenesulfonic acid, benzenesulfonic acid, and methanesulfonic acid, salts with alkali metals such as lithium, potassium, and sodium, salts with alkaline earth metals such as magnesium and calcium, and salts with an organic base such as ammonium salts. These salts can be formed by methods well known in the art.

For example, in the case where the compound of the present invention is a hydrochloride salt, the hydrochloride salt can be prepared by dissolving the azabenzimidazole compound according to the present invention in a solution of hydrogen chloride in alcohol, a solution of hydrogen chloride in ethyl acetate, a solution of hydrogen chloride in 1,4-dioxane, a solution of hydrogen chloride in cyclopentyl methyl ether, or a solution of hydrogen chloride in diethyl ether.

Some of the compounds of the present invention may have an asymmetric carbon, and the respective stereo isomers and mixtures thereof are all included in the present invention. The stereo isomers can be prepared, for example, by means of optical resolution from the racemate thereof according to a known method using an optically active acid (for example, tartaric acid, dibenzoyltartaric acid, mandelic acid, 10-camphor sulfonic acid, etc.), utilizing its basicity, or by using an optically active compound prepared in advance as a starting material. In addition, the stereo isomers may also be prepared by optical resolution using a chiral column or by asymmetric synthesis.

The formula [1] of the present invention is not limited to a specific isomer, but includes all possible isomers and racemates. For example, as shown below, tautomers [1Eq] and stereoisomers are also included.

[Chem. 15]

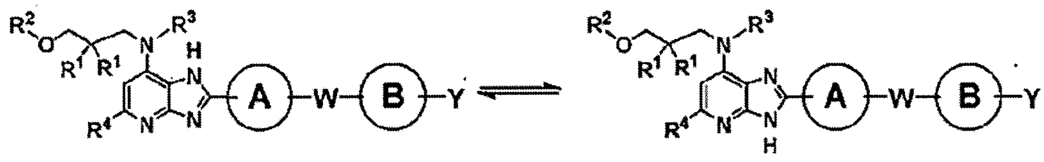

[1] [1Eq]

wherein the symbols are as defined above.

Acetylcholine (ACh) is a neurotransmitter that is released from the ends of the parasympathetic nerves and the motor nerves and that transmits nerve stimuli by binding to acetylcholine receptors (AChR). Acetylcholine receptors are roughly classified into G protein-coupled muscarinic receptors and ion channel type nicotinic receptors. Muscarinic receptors are classified into five subtypes, M1 to M5. Subtype M3 muscarinic receptors (hereinafter, sometimes referred to as "M3 receptors") have been reported to be mainly expressed in the bladder, gastrointestinal tract, pupil, salivary gland, lacrimal gland, etc., and be involved in contraction of the bladder, gastrointestinal tract, and pupil, secretion of saliva and tears, etc. (see NON-PATENT DOCUMENTS 4 and 5).

Regarding G protein-coupled receptors, there have been many reports on the structure of an allosteric site different from an orthosteric site to which an endogenous agonist binds, and this allosteric site is attracting much attention in recent years (see NON-PATENT DOCUMENT 6). Depending on the ligand that binds to the allosteric site, the structure of the receptor is changed, and the binding force between the endogenous agonist and the receptor is increased. Accordingly, endogenous agonist-stimulation-dependent signal levels can be enhanced for the receptor. As used herein, a ligand that enhances the signal level of the receptor due to the endogenous agonist by binding to the allosteric site as described above is referred to as a positive allosteric modulator (PAM). That is, a positive allosteric modulator means a ligand that binds to the allosteric site different from the orthosteric site, to which the endogenous agonist binds, and enhances a signal of the agonist.

Also, regarding M3 receptors, in recent years, an allosteric site different from an orthosteric site to which an endogenous agonist (acetylcholine, muscarinic) binds has been reported (see NON-PATENT DOCUMENT 7). M3 receptor PAMs (hereinafter, referred to as "M3 PAMs") are considered to be able to enhance endogenous agonist-stimulation-dependent signal levels for M3 receptors. Therefore, M3 PAMs can enhance the signal levels of M3 receptors under more physiological conditions, and are expected to be therapeutically promising for the treatment of diseases involving M3 receptors.

As shown in Test Examples described later, the compound of the present invention has M3 PAM activity and has an effect of improving gastrointestinal function and an effect of promoting salivation. Here, M3 PAM activity means an effect of enhancing M3 receptor function by binding to a site (allosteric site) different from the binding site (orthosteric site) of an endogenous activator (acetylcholine or muscarine) in the M3 receptor.

Therefore, the compound of the present invention can be used as a therapeutic agent or a prophylactic agent for functional gastrointestinal disorders or xerostomia.

When the compound of the present invention is administered as a pharmaceutical, the compound of the present invention is administered to a mammal including human as it is or as a pharmaceutical composition containing the compound in an amount, such as 0.001% to 99.5%, preferably 0.1% to 90%, in a pharmaceutically acceptable non-toxic and inert carrier.

The diseases to which the compound of the present invention can be applied include functional gastrointestinal disorders. Functional gastrointestinal disorders include functional esophageal disorders, functional gastroduodenal disorders, functional bowel disorders, functional abdominal pain syndrome, functional gallbladder/Oddi's sphincter disorders, and functional rectal-anal syndrome. For example, functional esophageal disorders include functional heartburn, functional gastroduodenal disorders include functional dyspepsia, and functional bowel disorders include irritable bowel syndrome (IBS), functional constipation, and opioid-induced constipation.

Also, the diseases to which the compound of the present invention can be applied include xerostomia. Examples of xerostomia include xerostomia caused by a predetermined disease, aging, salivary gland disorder due to irradiation, mental fatigue, or side effects during drug administration. Examples of the predetermined disease include autoimmune diseases, viral diseases, diabetes, anemia, hypernatremia, and renal disorders.

The compound of the present invention can be used as a therapeutic agent for various disorders as described above, for example, for mammals such as humans, mice, rats, rabbits, dogs, cats, cows, horses, pigs, and monkeys, as it is or by mixing the compound of the present invention with a pharmacologically acceptable carrier or the like to

prepare a pharmaceutical composition containing, for example, 0.001 % to 99.5% and preferably 0.1% to 90%, of the compound of the present invention.

The dose as a pharmaceutical is preferably adjusted taking into consideration the conditions such as age, weight, type and severity of disease of the patient, administration route, type of the compound of the present invention, whether or not it is a salt, and the type of the salt. In general, the effective amount of the compound of the present invention for adult, in the case of oral administration, is preferably within a range of 0.01 mg to 5 g/day/adult, preferably 1 mg to 500 mg/day/adult. In some cases, a smaller amount may be sufficient or a larger amount may be required. Usually, the dosage can be administered once a day or can be divided and administered several times a day, or in the case of intravenous administration, the dosage can be administered rapidly or sustainably within 24 hours.

One or more hydrogen, carbon, and/or the other atoms in the compound of the present invention may be replaced with an isotope thereof. Examples of such isotopes include $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$, and $^{36}Cl$, i.e., hydrogen, carbon, nitrogen, oxygen, phosphorus, sulfur, fluorine, iodine, and chlorine. The compound substituted with such an isotope is also useful as a pharmaceutical and includes all radiolabeled compounds of the compound of the present invention.

The present invention is described in more detail with reference to, but is not limited to, the following Reference Examples, Examples, and Test Examples.

The following abbreviations are used in the Examples.

TFA: Trifluoroacetic acid
Pt-C: Platinum-carbon
Pd-C: Palladium-carbon
$Pd_2(dba)_3 \bullet CHCl_3$: Tris(dibenzylideneacetone)bispalladium chloroform adduct
Pd2(dba)3: Tris(dibenzylideneacetone)bispalladium
$Pd(dppf)Cl_2 \bullet CH_2Cl_2$: [1,1'-Bis(diphenylphosphino)ferrocene]-dichloropalladium(II)•dichloromethane adduct
$Pd(OAc)_2$: Palladium(II) acetate
dppf: 1,1'-Bis(diphenylphosphino)ferrocene
XPhos: 2-Dicyclohexylphosphino-2',4',6'-triisopropylbiphenyl
RuPhos: 2-Dicyclohexylphosphino-2',6'-diisopropylbiphenyl
$PPh_3$: Triphenylphosphine
Boc: Tert-butoxycarbonyl
Bn: Benzyl
Ts: 4-Toluenesulfonyl
SEM: 2-(Trimethylsilyl)ethoxymethyl
DAST: (Diethylamino)sulfur-trifluoride
HATU: O-(7-azabenzotriazol-1-yl)-N,N,N',N'-tetramethyluronium hexafluorophosphate
DEAD: Diethyl azodicarboxylate
DMF: Dimethylformamide
DMSO: Dimethyl sulfoxide
THF: Tetrahydrofuran
DIPEA: N,N-diisopropylethylamine
TEA: Triethylamine
DBU: 1,8-Diazabicyclo[5.4.0]-7-undecene
$CDCl_3$: Deuterated chloroform
DMSO-d6: Deuterated dimethyl sulfoxide
TLC: Thin layer chromatography
MS: Mass spectrometry
LCMS: High performance liquid chromatography-Mass spectrometry
ESI: Electron Spray Ionization
M: Molar concentration (mol/L)

MS was performed using LCMS. ESI was used as a method for ionization. Observed values of the mass spectrometry are expressed as m/z.

The measurement conditions for LCMS are as follows.

Instrument: ACQUITY UPLC MS/PDA system (Waters)
Mass spectrometer: Waters 3100 MS detector
Photodiode array detector: ACQUITY PDA detector (UV-detected wavelength: 210 to 400 nm)
Column: Acquity BEH C18, 1.7 μm, 2.1x50 mm

Flow rate: 0.5 mL/min
Column temperature: 40°C
Solvent;
A: 0.1% formic acid/$H_2O$ (v/v; the same hereinafter)
B: 0.1% formic acid/acetonitrile

$^1H$ NMR spectrum was obtained using JNM-ECS400 Nuclear Magnetic Resonance Spectrometer (JEOL RESONANCE Ltd.). The observed peaks are shown as chemical shift values $\delta$ (ppm) (s = singlet, d = doublet, t = triplet, q = quartet, brs = broad singlet, m = multiplet, dd = double doublet, dt = double triplet).

In the experiment using microwave, Initiator 60 (manufactured by Biotage) was used, which can achieve a temperature of 40 to 250°C and a pressure of up to 20 bar.

The compounds described herein were named using naming software, ACD/NAME (registered trademark, Advanced Chemistry Development Inc.) according to IUPAC rules, or ChemBioDraw (version 14.0, Cambridge Soft), or named according to IUPAC nomenclature.

In a name of a compound, the descriptors "r" and "s" (lower case) refer to the stereochemistry of pseudoasymmetric carbon atom according to IUPAC rules.

Reference Example 1: 1-[1-(Ethoxymethl)cyclopentyl]-N-methylmethanamine hydrochloride

[Step 1] Preparation of 1-(ethoxymethyl)cyclopentane-1-carbonitrile

60% sodium hydride (16.5 g) was added to a solution of 1-(hydroxymethyl)cyclopentane-1-carbonitrile (43.1 g) in DMF (1150 mL) with stirring under ice-cooling, and the mixture was stirred at room temperature for 1 hour. Ethyl iodide (64.4 g) was added to the mixture under ice-cooling, and the mixture was stirred at room temperature. After confirming the consumption of the starting material on TLC, water and ethyl acetate were added to the reaction mixture, and the mixture was extracted with ethyl acetate. The organic layer was washed with water and saturated saline and dried over anhydrous sodium sulfate, and then the solvent was removed under reduced pressure. The residue was purified by silica gel column chromatography to afford the title compound (48.0 g).

[Step 2] Preparation of tert-butyl {[1-(ethoxymethyl)cyclopentyl]methyl}methyl carbamate

Lithium aluminum hydride (11.4 g) was suspended in THF (800 mL), and a solution of 1-(ethoxymethyl)cyclopentane-1-carbonitrile (46.0 g), obtained in Step 1, in THF (200 mL) was added dropwise to the mixture with stirring under ice-cooling. After the completion of the dropping, the mixture was stirred at room temperature for 2 hours. The reaction mixture was ice-cooled, and water (11.4 mL), 15% aq. sodium hydroxide (11.4 mL), and water (34.2 mL) were sequentially added dropwise to the reaction mixture. After the completion of the dropping, the mixture was stirred at room temperature for 2 hours, and the insolubles were filtered off through Celite and washed with THF (220 mL) three times. Triethylamine (46.0 mL) and di-tert-butyl dicarbonate (72.1 g) were added to the resulting filtrate with stirring at room temperature, and the mixture was stirred at the same temperature for 2 hours. The reaction mixture was concentrated under reduced pressure, and the residue was diluted with water and extracted with ethyl acetate. The organic layer was washed with saturated saline, dried over anhydrous sodium sulfate, and then concentrated under reduced pressure. The residue was dissolved in DMF (600 mL), 60% sodium hydride (14.4 g) was added to the solution with stirring under ice-cooling, and the mixture was stirred at room temperature for 1 hour. The reaction mixture was ice-cooled, and methyl iodide (22.5 mL) was added dropwise to the reaction mixture. After the completion of the dropping, the mixture was stirred at room temperature for 15 hours. The reaction mixture was ice-cooled, diluted with water, and then extracted with ethyl acetate-hexane (1:2). The organic layer was washed with saturated saline and dried over anhydrous sodium sulfate, and then the solvent was removed under reduced pressure. The residue was purified by silica gel column chromatography to afford the title compound (71.2 g).

[Step 3] Preparation of 1-[1-(ethoxymethyl)cyclopentyl]-N-methylmethanamine hydrochloride

A solution of tert-butyl {[1-(ethoxymethyl)cyclopentyl]methyl}methyl carbamate (71.2 g), obtained in Step 2, in ethyl acetate (52.5 mL) was stirred at room temperature, and hydrogen chloride (4 M in ethyl acetate, 328 mL) was added to the mixture, and the mixture was stirred at the same temperature for 2 hours. The reaction mixture was concentrated under reduced pressure, and the precipitated solid was collected by filtration, washed with hexane, and then dried to afford the title compound (50.3 g).

Reference Example 2: 1-[1-(Methoxymethyl]cyclopentyl]-N-methylmethanamine hydrochloride

[Step 1] Preparation of tert-butyl {[1-(hydroxymethyl)cyclopentyl]methyl} carbamate

Triethylamine (60.2 mL) was added to a solution of [1-(aminomethyl)cyclopentyl]methanol (50.7 g) in THF (304 mL) with stirring under ice-cooling. A solution of di-tert-butyl dicarbonate (94.2 g) in THF (101 mL) was added dropwise to the mixture. After the completion of the dropping, the mixture was stirred at room temperature overnight. The reaction mixture was diluted with water and ethyl acetate and extracted with ethyl acetate. The organic layer was washed with saturated saline and dried over anhydrous sodium sulfate, and then the solvent was removed under reduced pressure. The residue was diluted with ethyl acetate-hexane (1:9) (700 mL), and the dilution was stirred at room temperature for 3 hours. The insolubles were collected by filtration, washed with hexane, and then dried to afford the title compound (49.2 g). In addition, the solvent was removed under reduced pressure from the filtrate, and the residue was purified by silica gel column chromatography to afford the title compound (15.9 g).

[Step 2] Preparation of tert-butyl {[1-(methoxymethyl)cyclopentyl]methyl} methyl carbamate

Methyl iodide (47 mL) was added to a solution of tert-butyl {[1-(hydroxymethyl)cyclopentyl]methyl}carbamate (58 g), obtained in Step 1, in DMF (505 mL) with stirring at room temperature. Subsequently, 60% sodium hydride (30 g) was added portion-wise to the mixture with stirring under ice-cooling. The mixture was stirred under ice cooling for 30 minutes, then the temperature of the mixture was increased to room temperature, and the mixture was stirred overnight. Water (800 mL) was added dropwise to the reaction mixture under ice-cooling, and the mixture was extracted with ethyl acetate. The organic layer was washed with saturated saline and dried over anhydrous sodium sulfate, and then the solvent was removed under reduced pressure. The residue was purified by silica gel column chromatography to afford the title compound (68 g).

[Step 3] Preparation of 1-[1-(methoxymethyl)cyclopentyl]-N-methylmethanamine hydrochloride

The title compound (52 g) was obtained as described in Reference Example 1, Step 3, using tert-butyl {[1-(methoxymethyl)cyclopentyl]methyl}methyl carbamate obtained in Step 2 instead of tert-butyl {[1-(ethoxymethyl)cyclopentyl]methyl}methyl carbamate.

Reference Example 3: 4-Chloro-6-[3-fluoro-5-(trifluoromethyl)phenyl]pyridin-2-amine

1,4-Dioxane (9.6 mL) and water (2.4 mL) were added to [3-fluoro-5-(trifluoromethyl)phenyl]boronic acid (0.6 g), 4,6-dichloropyridin-2-amine (0.45 g), and potassium carbonate (1.2 g), and the mixture was degassed. Then, $Pd(dppf)Cl_2 \cdot CH_2Cl_2$ (118 mg) was added to the mixture at room temperature with stirring under an argon atmosphere, and the mixture was stirred at 80°C for 3 hours. The reaction mixture was cooled to room temperature, then diluted with water and ethyl acetate, and extracted with ethyl acetate. The organic layer was washed with water and saturated saline, dried over anhydrous magnesium sulfate, and then concentrated under reduced pressure. The residue was purified by silica gel column chromatography to afford the title compound (0.5 g).

Reference Example 4: 4-Chloro-6-[3-fluoro-5-(trifluoromethyl)phenyl]-3-nitropyridin-2-amine

Concentrated sulfuric acid (2.5 mL) was added to 4-chloro-6-[3-fluoro-5-(trifluoromethyl)phenyl]pyridin-2-amine (0.5 g) under ice-cooling, and then potassium nitrate (165 mg) was added portion-wise to the mixture. The mixture was stirred for 15 minutes under ice-cooling and then stirred at room temperature for 4 hours. The reaction mixture was poured into ice-water, 4 M aq. sodium hydroxide (25 mL) was added to the mixture, and then the mixture was extracted with ethyl acetate. The organic layer was washed with saturated saline, dried over anhydrous magnesium sulfate, and then concentrated under reduced pressure. The residue was purified by silica gel column chromatography to afford the title compound (0.35 g).

Reference Example 5: 6-Chloro-$N^4$-(3-methoxy-2,2-dimethylpropyl)-$N^4$-methyl-3-nitropyridin-2,4-diamine

A mixture of 4,6-dichloro-3-nitropyridin-2-amine (6.3 g), 3-methoxy-N,2,2-trimethylpropan-1-amine hydrochloride (6.6 g), DIPEA (16 mL), and 2-propanol (100 mL) was stirred at 60°C for 1 hour. The mixture was cooled to room temperature, water (50 mL) was added to the mixture, and the precipitate was collected by filtration, washed sequentially with 2-propanol and water, and then dried to afford the title compound (8.0 g).

Reference Example 6: 6'-Cyclopropyl-$N^4$-{[1-(methoxymethyl)cyclohexyl]methyl}-$N^4$-methyl-5-nitro-5'-(trifluoromethyl)[2,3'-bipyridine]-4,6-diamine

A mixture of 6-chloro-$N^4$-{[1-(methoxymethyl)cyclohexyl]methyl}-$N^4$-methyl-3-nitropyridin-2,4-diamine (2.5 g), 2-cyclopropyl-5-(4,4,5,5-tetramethyl-1,3,2-dioxaborolan-2-yl)-3-(trifluoromethyl)pyridine (2.7 g), potassium carbonate (3.0 g), 1,4-dioxane (29 mL), and water (11 mL) was degassed, and $Pd(dppf)Cl_2 \bullet CH_2Cl_2$ (0.24 g) was added to the mixture with stirring at room temperature under an argon atmosphere. The mixture was stirred at 95°C for 2 hours. The reaction mixture was cooled to room temperature, then diluted with water and ethyl acetate, and extracted with ethyl acetate. The organic layer was washed with water and saturated saline, dried over anhydrous magnesium sulfate, and then concentrated under reduced pressure. The residue was purified by silica gel column chromatography to afford the title compound (3.5 g).

Reference Example 7: 2'-Ethoxy-$N^4$-{[1-(methoxymethyl)cyclobutylmethyl}-$N^4$-methyl-6'-(trifluoromethyl)[2,4'-bipyridine]-4,5,6-triamine

Ammonium chloride (234 mg) and reduced iron (powder, 244 mg) were added to a mixture of 2'-ethoxy-$N^4$-{[1-(methoxymethyl)cyclobutyl]methyl}-$N^4$-methyl-5-nitro-6'-(trifluoromethyl)[2,4'-bipyridine]-4,6-diamine(684 mg), 2-propanol (7.5 mL), and water (2.5 mL) at room temperature, and the mixture was stirred at 90°C overnight. The reaction solution was cooled to room temperature and then diluted with ethyl acetate and water, and the insolubles were filtered off through Celite. The filtrate was extracted with ethyl acetate, and the organic layer was washed with saturated saline and then dried over anhydrous sodium sulfate. The solvent was removed under reduced pressure, and the residue was purified by silica gel column chromatography to afford the title compound (570 mg).

Reference Example 8: 6'-Cyclopropyl-$N^4$-{[1-(ethoxymethyl)cyclopentyl]methyl}-$N^4$-methyl-5'-(trifluoromethyl)[2,3'-bipyridine]1-4,5,6-triamine

Zinc powder (3.9 g) was added to a mixture of 6'-cyclopropyl-$N^4$-{[1-(ethoxymethyl)cyclopentyl]methyl)-$N^4$-methyl-5-nitro-5'-(trifluoromethyl)[2,3'-bipyridine]-4,6-diamine (5.8 g), ammonium chloride (1.9 g), 2-propanol (39 mL), and water (20 mL) with stirring at room temperature, and the mixture was stirred at 50°C for 4 hours. The reaction mixture was cooled to room temperature and then diluted with ethyl acetate, and the insolubles were filtered off through Celite. The filtrate was concentrated under reduced pressure, and then the residue was purified by silica gel column chromatography to afford the title compound (5.3 g).

Reference Example 9: Ethyl 3-[(3R)-4-(5-formylpyrazin-2-yl)-3-methylpiperazin-1-yl]propanoate

[Step 1] Preparation of tert-butyl (3R)-4-(5-formylpyrazin-2-yl)-3-methylpiperazine-1-carboxylate

A mixture of 5-chloropyrazine-2-carbaldehyde (350 mg), tert-butyl (3R)-3-methylpiperazine-1-carboxylate (541 mg), DIPEA (1.28 mL), and THF (4.9 mL) was stirred at 70°C for 3 hours. The reaction mixture was cooled to room temperature, diluted with water and ethyl acetate, and then extracted with ethyl acetate. The organic layer was washed with saturated saline and dried over anhydrous magnesium sulfate, and then the solvent was removed under reduced pressure. The residue was purified by silica gel column chromatography to afford the title compound (755 mg).

[Step 2] Preparation of ethyl 3-[(3R)-4-(5-formylpyrazin-2-yl)-3-methylpiperazin-1-yl] propanoate

Tert-butyl (3R)-4-(5-formylpyrazin-2-yl)-3-methylpiperazine-1-carboxylate (816 mg) was dissolved in ethyl acetate (5.3 mL), hydrogen chloride (4 M in ethyl acetate, 5.3 mL) was added to the solution with stirring at room temperature, and the mixture was stirred at the same temperature for 1 hour. The solvent was removed under reduced pressure, and the residue was mixed with acetonitrile (5 mL). DIPEA (2.31 mL) and ethyl 3-bromopropanoate (0.442 mL) were added to the mixture with stirring at room temperature, and the mixture was stirred at 70°C for 4 hours. The reaction mixture was cooled to room temperature and then purified by silica gel column chromatography to afford the title compound (676 mg).

Reference Example 10: Ethyl 1-(5-formylpyrazin-2-yl)piperidine-4-carboxylate

A mixture of 5-chloropyrazine-2-carbaldehyde (0.49 g), ethyl piperidine-4-carboxylate (0.54 g), DMSO (10 mL), and sodium bicarbonate (1.4 g) was stirred at 70°C for 17 hours. The mixture was cooled to room temperature, then ice-cooled, diluted with water, 2 M hydrochloric acid (6 mL), and ethyl acetate, and extracted with ethyl acetate. The

organic layer was washed with saturated saline and dried over anhydrous magnesium sulfate, and then the solvent was removed under reduced pressure. The residue was purified by silica gel column chromatography to afford the title compound (0.79 g).

Reference Example 11: Ethyl 3-[4-(5-formylpyrazin-2-yl)piperazin-1-yl]propanoate

A mixture of ethyl 3-(piperazin-1-yl)propanoate (0.142 mL), 5-chloropyrazine-2-carbaldehyde (100 mg), potassium carbonate (485 mg), and DMSO (3.5 mL) was stirred at 90°C for 2 hours. The reaction mixture was cooled to room temperature, then diluted with saturated aq. ammonium chloride, and extracted with ethyl acetate. The organic layer was washed with water and saturated saline and dried over anhydrous magnesium sulfate, and then the solvent was removed under reduced pressure. The residue was purified by silica gel column chromatography to afford the title compound (138 mg).

Reference Example 12: Ethyl {[1-(5-formylpyrazin-2-yl)piperidin-4-yl]oxy}acetate

A mixture of 5-chloropyrazine-2-carbaldehyde (0.55 g), ethyl [(piperidin-4-yl)oxy]acetate hydrochloride (0.92 g), THF (7.7 mL), and DIPEA (2.7 mL) was stirred at 70°C for 4 hours. The reaction mixture was cooled to room temperature, then diluted with saturated aq. ammonium chloride, and extracted with ethyl acetate. The organic layer was washed with water and saturated saline and dried over anhydrous sodium sulfate, and then the solvent was removed under reduced pressure. The residue was purified by silica gel column chromatography to afford the title compound (1.0 g).

Reference Example 13: 2'-cyclopropyl-$N^4$-[[1-(methoxymethyl)cyclohexyl]methyl}-$N^4$-methyl-6'-(trifluoromethyl)[2,4'-bipyridine]-4,5,6-triamine

A mixture of 6-chloro-$N^4$-{[1-(methoxymethyl)cyclohexyl]methyl}-$N^4$-methyl-3-nitropyridin-2,4-diamine (1.0 g), 2-cyclopropyl-4-(4,4,5,5-tetramethyl-1,3,2-dioxaborolan-2-yl)-6-(trifluoromethyl)pyridine (1.1 g), potassium carbonate (1.2 g), $Pd(dppf)Cl_2 \cdot CH_2Cl_2$ (95 mg), 1,4-dioxane (12 mL), and water (4.5 mL) was degassed and stirred at 90°C under an argon atmosphere for 2 hours. The reaction mixture was cooled to room temperature, then diluted with water and ethyl acetate, and extracted with ethyl acetate. The organic layer was washed with water and saturated saline, dried over anhydrous sodium sulfate, and then concentrated under reduced pressure. The residue was purified by silica gel column chromatography to afford 2'-cyclopropyl-$N^4$-{[1-(methoxymethyl)cyclohexyl]methyl}-$N^4$-methyl-5-nitro-6'-(trifluoromethyl)[[2,4'-bipyridine]-4,6-diamine. This compound was mixed with 2-propanol (9.7 mL), water (2.9 mL), ammonium chloride (0.47 g), and zinc powder (0.95 g), and the mixture was stirred at room temperature for 1 hour. The insolubles were filtered off through Celite, and the filtrate was concentrated under reduced pressure. The residue was purified by silica gel column chromatography to afford the title compound (1.2 g).

Reference Example 14: Ethyl [4-(3-fluoro-4-formylphenoxy)piperidin-1-yl]acetate

[Step 1] Preparation of tert-butyl 4-(3-fluoro-4-formylphenoxy)piperidine-1-carboxylate

$PPh_3$ (782 mg) was added to a mixture of tert-butyl 4-hydroxypiperidine-1-carboxylate (400 mg), 2-fluoro-4-hydroxybenzaldehyde (175 mg), and THF (10 mL) with stirring at room temperature, and DEAD (1.4 mL) was added to the mixture under ice-cooling. The temperature of the mixture was increased to room temperature, and the mixture was stirred. The reaction mixture was diluted with ethyl acetate and washed sequentially with saturated aq. sodium bicarbonate and saturated saline, and then the organic layer was dried over anhydrous magnesium sulfate. The solvent was removed under reduced pressure, and the residue was purified by silica gel column chromatography to afford the title compound (175 mg).

[Step 2] Preparation of ethyl [4-(3-fluoro-4-formylphenoxy)piperidin-1-yl]acetate

Hydrogen chloride (4 M in ethyl acetate, 0.68 mL) was added to a solution of tert-butyl 4-(3-fluoro-4-formylphenoxy)piperidine-1-carboxylate (175 mg), obtained in Step 1, in methanol (1.4 mL) with stirring at room temperature, and the mixture was stirred at the same temperature for 2 hours, stirred at 40°C for 2 hours, and stirred at room temperature overnight. The reaction mixture was concentrated under reduced pressure and then dried to afford 2-fluoro-4-[(piperidin-4-yl)oxy]benzaldehyde hydrochloride. This compound was mixed with acetonitrile (2 mL), DIPEA (0.47 mL) and ethyl bromoacetate (0.078 mL) were added to the mixture with stirring at room temperature, and the mixture was stirred at the same temperature for 3 hours. The reaction mixture was purified by silica gel column chromatography to afford the title compound (130 mg).

Reference Example 15: Ethyl [4-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperazin-1-yl]acetate

A mixture of 6-[3-fluoro-5-(trifluoromethyl)phenyl]-$N^4$-{[1-(methoxymethyl)cyclobutyl]methyl}-$N^4$-methylpyridine-2,3,4-triamine (50 mg), ethyl [4-(5-formylpyrazin-2-yl)piperazin-1-yl]acetate (35 mg), sodium dithionite (53 mg), and DMF (1 mL) was stirred at 100°C for 4 hours. Sodium dithionite (21 mg) was further added to the mixture, and the mixture was stirred at 100°C for 3 hours. The reaction mixture was cooled to room temperature and then diluted with saturated aq. sodium bicarbonate and water, and the resulting precipitate was collected by filtration, washed with water, and dried to afford the title compound (67 mg).

Reference Example 16: Ethyl 1-(5-{5-[2-ethoxy-6-(trifluoromethyl)pyridin-4-yl]-7-[{[1-(methoxymethyl)cyclobutyl]methyl}(methyl)amino]-1H-imidazo[4,5-b]pyridin-2-yl}pyrazin-2-yl)piperidine-4-carboxylate

A mixture of 2'-ethoxy-$N^4$-{[1-(methoxymethyl)cyclobutyl]methyl}-$N^4$-methyl-6'-(trifluoromethyl)[2,4'-bipyridine]-4,5,6-triamine (2.30 g), ethyl 1-(5-formylpyrazin-2-yl)piperidine-4-carboxylate (1.45 g), sodium dithionite (2.30 g), and DMF (26 mL) was stirred at 110°C for 4.5 hours. The reaction mixture was cooled to room temperature and then concentrated under reduced pressure. The residue was purified by silica gel column chromatography to afford the title compound (3.06 g).

Reference Example 17: Ethyl {[1-(5-{5-[2-cyclopropyl-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2 yl)piperidin-4-yl]oxy}acetate

A mixture of 2'-cyclopropyl-$N^4$-{[1-(methoxymethyl)cyclopentyl]methyl}-$N^4$-methyl-6'-(trifluoromethyl)[2,4'-bipyridine]-4,5,6-triamine (115 mg), ethyl {[1-(5-formylpyrazin-2-yl)piperidin-4-yl]oxy} acetate (79 mg), sodium dithionite (112 mg), and DMF (2.6 mL) was stirred at 100°C for 5 hours. The mixture was cooled to room temperature, then diluted with water and saturated aq. ammonium chloride, and extracted with ethyl acetate-hexane. The organic layer was washed with water and saturated saline, and the solvent was removed under reduced pressure. The residue was purified by silica gel column chromatography to afford the title compound (142 mg).

Reference Example 18: Ethyl 1-(5-15-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylate

A mixture of 2'-ethoxy-$N^4$-(3-methoxy-2,2-dimethylpropyl)-$N^4$-methyl-6'-(trifluoromethyl)[2,4'-bipyridine]-4,5,6-triamine (50 mg), ethyl 1-(5-formylpyrazin-2-yl)piperidine-4-carboxylate (32 mg), sodium dithionite (51 mg), and DMF (1 mL) was stirred at 100°C for 8 hours. The reaction mixture was cooled to room temperature and then concentrated under reduced pressure. The residue was purified by silica gel column chromatography to afford the title compound (75 mg).

Reference Example 19: Ethyl 1-(5-{5-[6-cyclopropyl-5-(trifluoromethyl)pyridin-3-yl]-7-[{[1-(ethoxymethyl)cyclopentyl]methyl](methyl)amino-1H-imidazo[4,5-b]pyridin-2-yl}pyrazin-2-yl)piperidine-4-carboxylate

A mixture of 6'-cyclopropyl-$N^4$-{[1-(ethoxymethyl)cyclopentyl]methyl}-$N^4$-methyl-5'-(trifluoromethyl)[2,3'-bipyridine]-4,5,6-triamine (50 mg), ethyl 1-(5-formylpyrazin-2-yl)piperidine-4-carboxylate (30 mg), sodium dithionite (23 mg), and DMF (0.72 mL) was stirred at 100°C for 8 hours. The reaction mixture was cooled to room temperature and then purified by silica gel column chromatography to afford the title compound (76 mg).

Reference Example 20: Ethyl 1-(5-{5-[2-ethoxy-6-(trifluoromethyl)pyridin-4-yl]-7-[{[1-(methoxymethyl)cyclopentyl]methyl}(methyl)amino]-1H-imidazo][4,5-b]pyridin-2-yl}pyrazin-2-yl)piperidine-4-carboxylate

A mixture of 2'-ethoxy-$N^4$-{[1-(methoxymethyl)cyclopentyl]methyl}-$N^4$-methyl-6'-(trifluoromethyl)[2,4'-bipyridine]-4,5,6-triamine (50 mg), ethyl 1-(5-formylpyrazin-2-yl)piperidine-4-carboxylate (31 mg), sodium dithionite (48 mg), and DMF (1 mL) was stirred at 100°C for 8 hours. The reaction mixture was cooled to room temperature and then concentrated under reduced pressure. The residue was purified by silica gel column chromatography to afford the title compound (75 mg).

Reference Example 21: Ethyl 3-[4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl)propanoate

A mixture of 6'-cyclopropyl-$N^4$-{[1-(methoxymethyl)cyclohexyl]methyl}-$N^4$-methyl-5'-(trifluoromethyl)[2,3'-bipyridine]-4,5,6-triamine (1.12 g), ethyl 3-[4-(5-formylpyrazin-2-yl)piperazin-1-yl]propanoate (0.742 g), sodium dithionite (1.05 g), and DMF (24 mL) was stirred at 110°C for 4 hours. The mixture was cooled to room temperature, then sodium dithionite (1.05 g) was further added to the mixture, and the mixture was stirred at 100°C for 3 hours. The mixture was cooled to room temperature, then diluted with water and saturated aq. sodium bicarbonate, and extracted with ethyl acetate. The organic layer was washed with water and saturated saline and dried over anhydrous magnesium sulfate, and then the solvent was removed under reduced pressure. The residue was purified by silica gel column chromatography to afford the title compound (1.24 g).

Reference Example 22: Ethyl [4-(4-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}-3-fluorophenoxy)piperidin-1-yl]acetate

A mixture of 6'-cyclopropyl-$N^4$-{[1-(methoxymethyl)cyclopentyl]methyl}-$N^4$-methyl-5'-(trifluoromethyl)[2,3'-bipyridine]-4,5,6-triamine (35.6 mg), ethyl [4-(3-fluoro-4-formylphenoxy)piperidin-1-yl]acetate (25.7 mg), sodium dithionite (34.5 mg), and DMF (0.79 mL) was stirred at 110°C overnight. The reaction mixture was cooled to room temperature and then purified by silica gel column chromatography to afford the title compound (40.7 mg).

Reference Example 23: Ethyl 3-[(2S)-4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)-2-(methoxymethyl)piperazin-1-yl]propanoate

A mixture of 6'-cyclopropyl-$N^4$-{[1-(methoxymethyl)cyclohexyl]methyl}-$N^4$-methyl-5'-(trifluoromethyl)[2,3'-bipyridine]-4,5,6-triamine (38 mg), ethyl 3-[(2S)-4-(5-formylpyrazin-2-yl)-2-(methoxymethyl)piperazin-1-yl]propanoate (29 mg), sodium dithionite (36 mg), and DMA (0.82 mL) was stirred at 110°C for 11 hours. The mixture was cooled to room temperature, then diluted with water, and extracted with dichloromethane. The organic layer was concentrated under reduced pressure, and then the residue was purified by silica gel column chromatography to afford the title compound (48 mg).

Reference Example 24: Ethyl 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoate

A mixture of 6'-cyclopropyl-$N^4$-{[1-(ethoxymethyl)cyclopentyl]methyl}-$N^4$-methyl-5'-(trifluoromethyl)[2,3'-bipyridine]-4,5,6-triamine (1.5 g), ethyl 3-[(3R)-4-(5-formylpyrazin-2-yl)-3-methylpiperazin-1-yl]propanoate (1.1 g), sodium dithionite (1.1 g), and DMF (15 mL) was stirred at 100°C for 3 hours. The reaction mixture was cooled to room temperature, then diluted with water and ethyl acetate, and extracted with ethyl acetate. The organic layer was washed with saturated saline and dried over anhydrous sodium sulfate, and then the solvent was removed under reduced pressure. The residue was purified by silica gel column chromatography to afford the title compound (1.9 g).

Reference Example 25: Ethyl 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoate

A mixture of 2' -cyclopropyl-$N^4$-{[1-(methoxymethyl)cyclohexyl]methyl}-$N^4$-methyl-6'-(trifluoromethyl)[2,4'-bipyridine]-4,5,6-triamine (50 mg), ethyl 3-[(3R)-4-(5-formylpyrazin-2-yl)-3-methylpiperazin-1-yl]propanoate (36 mg), sodium dithionite (47 mg), and DMF (0.5 mL) was stirred at 100°C for 8 hours. The reaction mixture was cooled to room temperature and then concentrated under reduced pressure. The residue was purified by silica gel column chromatography to afford the title compound (76 mg).

Example 1: [4-(5-{5-[3-Fluoro-5-(trifluoromethyl)phenyl]-7-[{[1-(methoxymethyl)cyclobutylmethyl}(methyl)amino]-1H-imidazo[4,5-b]pyridin-2-yl}pyrazin-2-yl)piperazin-1-yl]acetic acid

1 M aq. sodium hydroxide (0.134 mL) was added to a solution of ethyl [4-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperazin-1-yl]acetate (67 mg) in ethanol (1 mL) with stirring at room temperature, and the mixture was stirred at the same temperature for 4 hours. The reaction mixture was diluted with water and neutralized with 2 M hydrochloric acid. The resulting precipitate was collected by filtration, washed with water, and then dried to afford the title compound (60 mg).

Example 2: 4-Fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylic acid

The title compound (67 mg) was obtained as described in Example 1, using ethyl 4-fluoro-1-(5-{5-[3-fluoro-5-(trifluoromethyl)phenyl]-7-[{[1-(methoxymethyl)cyclobutyl]methyl} (methyl)amino]-1H-imidazo[4,5-b]pyridin-2-yl} pyrazin-2-yl)piperidine-4-carboxylate (77 mg) instead of ethyl [4-(5-{ 5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperazin-1-yl]acetate.

Example 3: 1-(5-{5-[6-Ethoxy-5-(trifluoromethyl)pyridin-3-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid

1 M aq. sodium hydroxide (0.30 mL) was added to a solution of ethyl 1-(5-{5-[6-ethoxy-5-(trifluoromethyl)pyridin-3-yl]-7-[{[1-(methoxymethyl)cyclobutyl]methyl} (methyl)amino]-1H-imidazo[4,5-b]pyridin-2-yl} pyrazin-2-yl)piperidine-4-carboxylate (41 mg) in ethanol (1 mL), and the mixture was stirred at 70°C for 2 hours. The mixture was cooled to room temperature, then diluted with water, and neutralized with 1 M hydrochloric acid. The resulting precipitate was collected by filtration and dried to afford the title compound (30 mg).

Example 4: 1-(5-{5-[2-Ethoxy-6-(trifluoromethyl)pyridin-4-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid dihydrochloride

[Step 1] Preparation of 1-(5-{5-[2-ethoxy-6-(trifluoromethyl)pyridin-4-yl]-7-[{[1-(methoxymethyl)cyclobutyl]methyl} (methyl)amino]-1H-imidazo[4,5-b]pyridin-2-yl}pyrazin-2-yl)piperidine-4-carboxylic acid

A mixture of ethyl 1-(5-{5-[2-ethoxy-6-(trifluoromethyl)pyridin-4-yl]-7-[{[1-(methoxymethyl)cyclobutyl]methyl} (methyl)amino]-1H-imidazo[4,5-b]pyridin-2-yl}pyrazin-2-yl)piperidine-4-carboxylate (285 mg), ethanol (8 mL), and 1 M aq. sodium hydroxide (2.1 mL) was stirred at 50°C for 1 hour. The mixture was cooled to room temperature, then the solvent was concentrated under reduced pressure, and the residue was diluted with water and neutralized with 1 M hydrochloric acid. The resulting precipitate was collected by filtration and dried to afford the title compound (245 mg).

[Step 2] Preparation of 1-(5-{5-[2-ethoxy-6-(trifluoromethyl)pyridin-4-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid dihydrochloride

1-(5-{5-[2-Ethoxy-6-(trifluoromethyl)pyridin-4-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid (245 mg) obtained in Step 1 was diluted with ethyl acetate (8 mL), hydrogen chloride (4M in ethyl acetate, 0.47 mL) was added to the dilution with stirring at room temperature, and the mixture was stirred at the same temperature for 1 hour. The solvent was removed under reduced pressure, the residue was diluted with diethyl ether, and the insolubles were collected by filtration, washed with diethyl ether, and then dried to afford the title compound (244 mg).

Example 5: {[1-(5-{5-[2-Cyclopropyl-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidin-4-yl]oxy}acetic acid

Lithium hydroxide monohydrate (32.8 mg) was added to a mixture of ethyl {[1-(5-{5-[2-cyclopropyl-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidin-4-yl]oxy}acetate (141 mg), THF (0.78 mL), methanol (0.78 mL), and water (0.78 mL), and the mixture was stirred at room temperature for 30 minutes and then stirred at 50°C overnight. The reaction mixture was cooled to room temperature and then concentrated under reduced pressure, and the residue was diluted by adding water thereto. The dilution was neutralized by adding 2 M hydrochloric acid thereto with stirring at room temperature. The resulting precipitate was collected by filtration, washed with water, and then dried to afford the title compound (133 mg).

Example 6: 1-(4-{5-[6-Ethoxy-5-(trifluoromethyl)pyridin-3-yl)-7-[{[1-(methoxymethyl)cyclopentyl]methyl}(methyl)amino]-1H-imidazo[4,5-b]pyridin-2-yl}-3-fluorophenyl)piperidine-4-carboxylic acid

Lithium hydroxide monohydrate (15 mg) was added to a mixture of ethyl 1-(4-{5-[6-ethoxy-5-(trifluoromethyl)pyridin-3-yl]-7-[{[1-(methoxymethyl)cyclopentyl]methyl}(methyl)amino]-1H-imidazo[4,5-b]pyridin-2-yl}-3-fluorophenyl)piperidine-4-carboxylate (58 mg), THF (0.81 mL), methanol (0.81 mL), and water (0.81 mL), and the mixture was stirred at 50°C overnight. The reaction mixture was cooled to room temperature and then concentrated under reduced pressure, and the residue was diluted by adding water thereto. The dilution was neutralized by adding 2 M hydrochloric acid thereto

with stirring at room temperature. The resulting precipitate was collected by filtration, washed with water, and then dried to afford the title compound (53 mg).

Example 7: 1-(5-{5-[2-Ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylic acid

1 M aq. sodium hydroxide (0.56 mL) was added to a solution of ethyl 1-(5-{5-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylate (75 mg) in ethanol (1 mL), and the mixture was stirred at 50°C for 2 hours. The mixture was cooled to room temperature, then diluted with water, and neutralized with 1 M hydrochloric acid. The resulting precipitate was collected by filtration and dried to afford the title compound (62 mg).

Example 8: 1-(5-{5-[6-Cyclopropyl-5-trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)piperidine-4-carboxylic acid

Lithium hydroxide monohydrate (23 mg) was added to a mixture of ethyl 1-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)piperidine-4-carboxylate (76 mg), ethanol (0.54 mL), THF (0.54 mL), and water (0.18 mL), and the mixture was stirred at room temperature for 2 hours. The solvent was removed under reduced pressure, and the residue was diluted with water and then neutralized by adding 6 M hydrochloric acid thereto. The resulting precipitate was collected by filtration to afford the title compound (40 mg).

Example 9: 1-(5-{5-[2-Ethoxy-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidine-4-carboxylic acid

1 M aq. sodium hydroxide (0.54 mL) was added to a solution of ethyl 1-(5-{5-[2-ethoxy-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidine-4-carboxylate (75 mg) in ethanol (1 mL), and the mixture was stirred at 50°C for 2 hours. The mixture was cooled to room temperature, then diluted with water, and neutralized with 1 M hydrochloric acid. The resulting precipitate was collected by filtration and dried to afford the title compound (63 mg).

Example 10: 3-[4-(5-{5-[6-Cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl]propanoic acid

Lithium hydroxide monohydrate (0.285 g) was added to a mixture of ethyl 3-[4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl]propanoate (1.24 g), THF (8.4 mL), methanol (8.4 mL), and water (8.4 mL) with stirring at room temperature, and the mixture was stirred at 50°C overnight. The reaction mixture was cooled to room temperature and then concentrated under reduced pressure. The residue was diluted with water, and neutralized by adding 2 M hydrochloric acid (3.4 mL) thereto with stirring at room temperature. The resulting precipitate was collected by filtration, washed with water, and then dried to afford the title compound (1.14 g).

Example 11: [4-(4-{5-[6-Cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}-3-fluorophenoxy)piperidin-1-yl]acetic acid

2 M aq. sodium hydroxide (0.134 mL) was added to a solution of ethyl [4-(4-{5-[6-cyclopropyl-5-(trifluoromethyl)pyridin-3-yl]-7-[{[1-(methoxymethyl)cyclopentyl]methyl} (methyl)amino]-1H-imidazo[4,5-b]pyridin-2-yl} -3-fluorophenoxy)piperidin-1-yl]acetate (39.5 mg) in ethanol (1.1 mL) with stirring at room temperature, and the mixture was stirred at the same temperature for 4 hours. The reaction mixture was diluted with water and neutralized with 2 M hydrochloric acid. The resulting precipitate was collected by filtration, washed with water, and then dried to afford the title compound (35.6 mg).

Example 12: 3-((2S)-4-(5-{5-[6-Cyclopropyl-5-(trifluoromethyl)pyridin-3-yl]-7-[{[1-(methoxymethyl)cyclohexyl)methyl}(methyl)amino]-1H-imidazo[4,5-b]pyridin-2-yl}pyrazin-2-yl)-2-(methoxymethyl)piperazin-1-yl]propanoic acid

Lithium hydroxide monohydrate (21.8 mg) was added to a mixture of ethyl 3-[(2S)-4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)-2-(methoxymethyl)piperazin-1-yl]propanoate (96.7 mg), THF (0.74 mL), methanol (0.74 mL), and water

(0.74 mL) with stirring at room temperature, and the mixture was stirred at the same temperature for 2 hours. The reaction mixture was concentrated under reduced pressure, and the residue was diluted with water and then neutralized with 2 M hydrochloric acid. The resulting precipitate was collected by filtration, washed with water, and then dried to afford the title compound (76.3 mg).

Example 13: Sodium 3-[(3R)-4-(5-{5-[6-cyclopropyl-5-(trifluoromethyl)pyridin-3-yl]-7-({[1-(ethoxymethyl)cyclopentyl]methyl}(methyl)amino]-1H-imidazo[4,5-b]pyridin-2-yl}pyrazin-2-yl)-3-methylpiperazin-1-yl]propanoate

[Step 1] Preparation of 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoic acid

4 M aq. sodium hydroxide (3 mL) was added to a mixture of ethyl 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoate (1.82 g), ethanol (9 mL), and water (9 mL) with stirring at room temperature, and the mixture was stirred at 50°C for 1 hour. The reaction mixture was diluted with water and neutralized with 6 M hydrochloric acid. The reaction mixture was stirred at room temperature overnight. Then, the resulting precipitate was collected by filtration, washed with water, and then dried to afford the title compound (1.73 g).

[Step 2] Preparation of sodium 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoate

Sodium methoxide (0.5 M in methanol, 3.32 mL) was added to a mixture of 3-[(3R)-4-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)-3-methylpiperazin-1-yl]propanoic acid (1.20 g) obtained in Step 1 and methanol (30 mL) with stirring at room temperature, and the mixture was stirred at the same temperature for 1 hour. The solvent was removed under reduced pressure, and the residue was diluted with diethyl ether (20 mL) and stirred at room temperature for 3 hours. Hexane (20 mL) was added to the dilution, and the mixture was stirred at room temperature for 1 hour. The insolubles were collected by filtration, washed with diethyl ether-hexane (1:1), and then dried to afford the title compound (1.16 g).

Example 14: Sodium 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3 -methylpiperazin-1-yl]propanoate

[Step 1] Preparation of 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid

1 M aq. sodium hydroxide (0.50 mL) was added to a solution of ethyl 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoate (76 mg) in ethanol (1 mL) with stirring at room temperature, and the mixture was stirred at the same temperature for 1 hour. The reaction mixture was neutralized by adding 2 M hydrochloric acid thereto and concentrated under reduced pressure, and then the residue was diluted with water. The resulting precipitate was collected by filtration, washed with water, and then dried to afford the title compound (68 mg).

[Step 2] Preparation of sodium 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoate

Sodium methoxide (0.5 M in methanol, 0.19 mL) was added to a mixture of 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid (68 mg) obtained in Step 1 and methanol (1 mL) with stirring at room temperature, and the mixture was stirred at the same temperature for 1 hour. The solvent was removed under reduced pressure, and the residue was diluted with diethyl ether-hexane (1:1) and then stirred at room temperature for 1 hour. The insolubles were collected by filtration, washed with diethyl ether-hexane (1:1), and then dried to afford the title compound (63 mg).

Example 15: Sodium 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoate

[Step 1] Preparation of ethyl 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoate

A mixture of 6'-cyclopropyl-$N^4$-{[1-(methoxymethyl)cyclopentyl]methyl}-$N^4$-methyl-5'-(trifluoromethyl)[2,3'-bipyridine]-4,5,6-triamine (50 mg), ethyl 3-[(3R)-4-(5-formylpyrazin-2-yl)-3-methylpiperazin-1-yl]propanoate (38 mg), sodium dithionite (39 mg), and DMF (0.5 mL) was stirred at 110°C for 3 hours. The reaction mixture was cooled to room temperature and then purified by silica gel column chromatography to afford the title compound (75 mg).

[Step 2] Preparation of 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoic acid

4 M aq. sodium hydroxide (0.127 mL) was added to a mixture of ethyl 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoate (75 mg) obtained in Step 1, THF (0.5 mL), and water (0.5 mL) with stirring at room temperature, and the mixture was stirred at 50°C for 1 hour. The reaction mixture was cooled to room temperature, then diluted with water, and neutralized by adding 1 M hydrochloric acid thereto. The resulting precipitate was collected by filtration, washed with water, and dried to afford the title compound (66 mg).

[Step 3] Preparation of sodium 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoate

Sodium methoxide (0.5 M in methanol, 0.184 mL) was added to a mixture of 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoic acid (65 mg) obtained in Step 2 and methanol (2 mL) with stirring at room temperature, and the mixture was stirred at the same temperature for 30 minutes. The solvent was removed under reduced pressure, and the residue was diluted with diethyl ether-hexane (1:1) and then stirred at room temperature for 1 hour. The insolubles were collected by filtration, washed with diethyl ether-hexane (1:1), and then dried to afford the title compound (65 mg).

Compounds of Reference Examples and Examples are further provided below in Tables 1 to 10. In the tables, PREx means the Reference Example No. where the compound was prepared according to the method as described in said Reference Example using a corresponding starting material. For example, the compound of the following Reference Example with the indication of PREx No. as 1 was prepared using the method as described in Reference Example 1. In addition, in the tables, Chemical Name refers to the name of the compound corresponding to the number of the Reference Example (REx) or the Example (Ex), and Data refers to the instrumental analytical data of the compound, such as mass spectrometric data (m/z values), $^1$H NMR data (δ (ppm) of peaks), and elemental analytical data (composition (%) of C, H, and N).

[Table 1]

| REx | PREx | Compound Name | Data |
|---|---|---|---|
| 1 | 1 | 1-[1-(Ethoxymethyl)cyclopentyl]-N-methylmethanamine hydrochloride | $^1$H-NMR (400 MHz, DMSO-$d_6$) δ: 3.58 (dd, 2H), 3.44 (s, 2H), 3.02 (t, 2H), 2.74 (t, 3H), 1.76-1.58 (m, 8H), 1.21 (t, 3H) |
| 2 | 2 | 1-[1 -(Methoxymethyl)cyclopentyl] -N-methylmethanamine hydrochloride | $^1$H-NMR (400 MHz, $CDCl_3$) δ: 3.42 (s, 3H), 3.41 (s, 2H), 3.02-2.99 (m, 2H), 2.75 (t, 3H), 2.17-2.12 (m, 2H), 1.75-1.56 (m, 6H) |
| 3 | 3 | 4-Chloro-6-[3-fluoro- 5-(trifluoromethyl)phenyl]pyridin-2-amine | MS (ESI+) m/z 291.0 (M+H)$^+$ |
| 4 | 4 | 4-Chloro-6-[3-fluoro-5-(trifluoromethyl)phenyl]-3-nitropyridin- 2-amine | MS (ESI+) m/z 235.9 (M+H)$^+$ |
| 5 | 5 | 6-Chloro-$N^4$-(3-methoxy-2,2-dimethylpropyl)-$N^4$-methyl-3-nitropyridin-2,4-diamine | MS (ESI+) m/z 303.6 (M+H)$^+$ |

(continued)

| REx | PREx | Compound Name | Data |
|---|---|---|---|
| 6 | 6 | 6'-Cyclopropyl-$N^4$-{[1-(methoxymethyl)cyclohexyl] methyl}-$N^4$-methyl-5-nitro-5'-(trifluoromethyl)[2,3'-bipyridine]-4,6-diamine | MS (ESI+) m/z 494.4 $(M+H)^+$ |
| 7 | 7 | 2'-Ethoxy-$N^4$-{[1-(methoxymethyl)cyclobutyl] methyl}-$N^4$-methyl-6'-(trifluoromethyl)[2,4'-bipyridine]-4,5,6-triamine | MS (ESI+) m/z 440.2 $(M+H)^+$ |

[Table 2]

| REx | PREx | Compound Name | Data |
|---|---|---|---|
| 8 | 8 | 6'-Cyclopropyl-N4-{[1-(ethoxymethyl)cyclopentyl]methyl}-N4-methyl-5'-(trifluoromethyl)[2,3'-bipyridine]-4,5,6-triamine | MS (ESI+) m/z 464.3 $(M+H)^+$ |
| 9 | 9 | Ethyl 3-[(3R)-4-(5-formylpyrazin-2-yl)-3-methylpiperazin-1-yl]propanoate | MS (ESI+) m/z 307.1 $(M+H)^+$ |
| 10 | 10 | Ethyl 1-(5-formylpyrazin-2-yl)piperidine-4-carboxylate | MS (ESI+) m/z 264.2 $(M+H)^+$ |
| 11 | 11 | Ethyl 3-[4-(5-formylpyrazin-2-yl)piperazin-1-yl]propanoate | MS (ESI+) m/z 293.5 $(M+H)^+$ |
| 12 | 12 | Ethyl {[1-(5-formylpyrazin-2-yl)piperidin-4-yl]oxy} acetate | MS (ESI+) m/z 294.1 $(M+H)^+$ |
| 13 | 13 | 2'-Cyclopropyl-$N^4$-{[1-(methoxymethyl)cyclohexyl]methyl} - $N^4$-methyl-6'-(trifluoromethyl)[2,4'-bipyridine]-4,5,6-triamine | MS (ESI+) m/z 464.8 $(M+H)^+$ |
| 14 | 14 | Ethyl [4-(3-fluoro-4-formylphenoxy)piperidin-1-yl]acetate | MS (ESI+) m/z 310.2 $(M+H)^+$ |

[Table 3]

| REx | PREx | Compound Name | Data |
|---|---|---|---|
| 15 | 15 | Ethyl [4-(5-{5-[3-fluoro-5-(trifluoromethyl)phenyl]-7-[{[1-(methoxymethyl) cyclobutyl]methyl}(methyl)amino]-1H-imidazo[4,5-b]pyridin-2-yl} pyrazin-2-yl) piperazin-1-yl]acetate | MS (ESI+) m/z 671.3 $(M+H)^+$ |
| 16 | 16 | Ethyl 1-(5-{5-[2-ethoxy-6-(trifluoromethyl)pyridin-4-yl]-7-[{[1-(methoxymethyl) cyclobutyl]methyl}(methyl)amino]-1H-imidazo[4,5-b]pyridin-2-yl}pyrazin-2-yl) pi peridine-4-carboxylate | MS (ESI+) m/z 683.9 $(M+H)^+$ |
| 17 | 17 | Ethyl {[1-(5-{5-[2-cyclopropyl-6-(trifluoromethyl)pyridin-4-yl]-7-[{ [1-(methoxymethyl)cyclopentyl]methyl}(methyl)amino]-1H-imidazo[4,5-b] pyridin-2-yl}pyrazin-2-yl)piperidin-4-yl]oxy}acetate | MS (ESI+) m/z 724.4 $(M+H)^+$ |
| 18 | 18 | Ethyl 1 -(5-{5-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl) piperidine-4-carboxylate | MS (ESI+) m/z 671.9 $(M+H)^+$ |

[Table 4]

| REx | PREx | Compound Name | Data |
|---|---|---|---|
| 19 | 19 | Ethyl 1-(5-{ 5-[6-cyclopropyl-5-(trifluoromethyl)pyridin-3-yl]-7-[{ [1-(ethoxymethyl)cyclopentyl]methyl} (methyl)amino]-1H-imidazo [4,5-b] pyridin-2-yl}pyrazin-2-yl)piperidine-4-carboxylate | MS (ESI+) m/z 708.0 $(M+H)^+$ |
| 20 | 20 | Ethyl 1-(5-{5-[2-ethoxy-6-(trifluoromethyl)pyridin-4-yl]-7-[{[1-(methoxymethyl) cyclopentyl]methyl}(methyl)amino]-1H-imidazo[4,5-b]pyridin-2-yl} pyrazin-2-yl)piperidine-4-carboxylate | MS (ESI+) m/z 697.9 $(M+H)^+$ |
| 21 | 21 | Ethyl 3-[4-(5-{5-[6-cyclopropyl-5-(trifluoromethyl)pyridin-3-yl]-7-[{ [1-(methoxymethyl)cyclohexyl]methyl} (methyl)amino]-1H-imidazo[4,5-b] pyridin-2-yl}pyrazin-2-yl)piperazin-1 -yl]propanoate | MS (ESI+) m/z 736.6 $(M+H)^+$ |
| 22 | 22 | Ethyl [4-(4- {5-[6-cyclopropyl-5-(trifluoromethyl)pyridin-3-yl]-7-[{ [1-(methoxymethyl)cyclopentyl]methyl}(methyl)amino]-1H-imidazo[4,5-b] pyridin-2-yl)-3-fluorophenoxy)piperidin-1-yl]acetate | MS (ESI+) m/z 740.2 $(M+H)^+$ |

[Table 5]

| REx | PREx | Compound Name | Data |
|---|---|---|---|
| 23 | 23 | Ethyl 3-[(2S)-4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)-2-(methoxymethyl) piperazin-1 -yl]propanoate | MS (ESI+) m/z 780.5 $(M+H)^+$ |
| 24 | 24 | Ethyl 3-[(3R)-4-(5-{5-[6-cyclopropyl-5-(trifluoromethyt)pyridin-3-yl]-7-[{[1-(ethoxymethyl)cyclopentyl]methyl} (methyl)amino]-1H-imidazo[4,5-b]pyridin-2-yl} pyrazin-2-yl)-3 -methylpiperazin-1 -yl]propanoate | MS (ESI+) m/z 751.0 $(M+H)^+$ |
| 25 | 25 | Ethyl 3-[(3 R)-4-(5- { 5-[2-cyclopropyl-6-(trifluoromethyl)pyridin-4-yl]-7-[{[1-(methoxymethyl)cyclohexyl]methyl} (methyl) amino]-1H-imidazo[4,5-b]pyridin-2-yl}pyrazin-2-yl)-3-methylpiperazin-1 -yl]propanoate | MS (ESI+) m/z 751.0 $(M+H)^+$ |
| 26 | 2 | 1-[1-(Methoxymethyl)cyclohexyl]-N-methylmethanamine hydrochloride | $^1$H-NMR (400 MHz, DMSO-d6) δ: 9.16 (brs, 2H), 3.49 (s, 2H), 3.41 (s, 3H), 2.94 (s, 2H), 2.74 (s, 3H), 1.62-1.39 (m, 10H) |

[Table 6]

| REx | PREx | Compound Name | Data |
|---|---|---|---|
| 27 | 5 | 6-Chloro-$N^4$-{[1-(methoxymethyl)cyclobutyl]methyl}-$N^4$-methyl-3-nitropyridin-2,4-diamine | MS (ESI+) m/z 315.5 $(M+H)^+$ |
| 28 | 5 | 6-Chloro-$N^4$-{[1-(methoxymethyl)cyclopentyl]methyl} - $N^4$-methyl-3-nitropyridin-2,4-diamine | MS (ESI+) m/z 329.1 $(M+H)^+$ |
| 29 | 5 | 6-Chloro-$N^4$-{[1-(methoxymethyl)cyclohexyl]methyl} - $N^4$-methyl-3-nitropyridin-2,4-diamine | MS (ESI+) m/z 343.5 $(M+H)^+$ |
| 30 | 5 | 6-Chloro-$N^4$-{[1-(ethoxymethyl)cyclopentyl]methyl}-$N^4$-methyl-3-nitropyridin-2,4-diamine | MS (ESI+) m/z 343.2 $(M+H)^+$ |
| 31 | 6 | 6-[3-Fluoro-5-(trifluoromethyl)phenyl]-$N^4$-{[1-(methoxymethyl)cyclobutyl] methyl}-$N^4$-methyl-3-nitropyridin-2,4-diamine | MS (ESI+) m/z 443.6 $(M+H)^+$ |

(continued)

| REx | PREx | Compound Name | Data |
|---|---|---|---|
| 32 | 6 | 6'-Ethoxy-$N^4$-{[1-(methoxymethyl)cyclobutyl]methyl}-$N^4$-methyl-5-nitro-5'-(trifluoromethyl)[2,3'-bipyridine]-4,6-diamine | MS (ESI+) m/z 470.2 $(M+H)^+$ |
| 33 | 6 | 6'-Ethoxy-$N^4$-{[1-(methoxymethyl)cyclopentyl]methyl}-$N^4$-methyl-5-nitro-5'-(trifluoromethyl)[2,3'-bipyridine] -4,6-diamine | MS (ESI+) m/z 484.1 $(M+H)^+$ |

[Table 7]

| REx | PREx | Compound Name | Data |
|---|---|---|---|
| 34 | 6 | 2'-Ethoxy-$N^4$-(3-methoxy-2,2-dimethylpropyl)-$N^4$-methyl-5-nitro-6'-(trifluoromethyl)[[2,4'-bipyridine]-4,6-diamine | MS (ESI+) m/z 458.7 $(M+H)^+$ |
| 35 | 6 | 2'-Ethoxy-$N^4$-{[1-(methoxymethyl)cyclobutyl]methyl}-$N^4$-methyl-5-nitro-6'-(trifluoromethyl)[[2,4' -bipyridine] - 4,6-diamine | MS (ESI+) m/z 470.2 $(M+H)^+$ |
| 36 | 6 | 2'-Ethoxy-$N^4$-{[1-(methoxymethyl)cyclopentyl]methyl}-$N^4$-methyl-5-nitro-6'-(trifluoromethyl)[[2,4' -bipyridine] - 4,6-diamine | MS (ESI+) m/z 484.7 $(M+H)^+$ |
| 37 | 6 | 6'-Cyclopropyl-$N^4$-{[1-(methoxymethyl)cyclopentyl]methyl} - $N^4$-methyl-5-nitro-5'-(trifluoromethyl)[2,3'-bipyridine]-4,6-diamine | MS (ESI+) m/z 480.6 $(M+H)^+$ |
| 38 | 6 | 6'-Cyclopropyl-$N^4$-{[1-(ethoxymethyl)cyclopentyl]methyl} - $N^4$-methyl-5 -nitro-5'-(trifluoromethyl) [2,3' -bipyridine]-4,6-diamine | MS (ESI+) m/z 494.3 $(M+H)^+$ |
| 39 | 6 | 2'-Cyclopropyl-$N^4$-{[1-(methoxymethyl)cyclopentyl]methyl} - $N^4$-methyl-5-nitro-6'-(trifluoromethyl)[[2,4'-bipyridine]-4,6-diamine | MS (ESI+) m/z 480.6 $(M+H)^+$ |
| 40 | 8 | 2' -Ethoxy-$N^4$-(3-methoxy-2,2-dimethylpropyl)-$N^4$-methyl-6'-(trifluoromethyl) [2,4' -bipyridine] - 4,5,6-triamine | MS (ESI+) m/z 428.4 $(M+H)^+$ |

[Table 8]

| REx | PREx | Compound Name | Data |
|---|---|---|---|
| 41 | 8 | 2'-Ethoxy-$N^4$-{[1-(methoxymethyl)cyclopentyl]methyl}-$N^4$-methyl-6'-(trifluoromethyl)[2,4'-bipyridine] -4,5,6-triamine | MS (ESI+) m/z 454.4 $(M+H)^+$ |
| 42 | 8 | 6'-Cyclopropyl-$N^4$-{[1-(methoxymethyl)cyclopentyl]methyl} - $N^4$-methyl-5'-(trifluoromethyl)[2,3'-bipyridine]-4,5,6-triamine | MS (ESI+) m/z 450.6 $(M+H)^+$ |
| 43 | 8 | 6'-Cyclopropyl-$N^4$-{[1-(methoxymethyl)cyclohexyl]methyl}-$N^4$-methyl-5'-(trifluoromethyl)[2,3'-bipyridine]-4,5,6-triamine | MS (ESI+) m/z 464.5 $(M+H)^+$ |
| 44 | 8 | 2'-Cyclopropyl-$N^4$-{[1-(methoxymethyl)cyclopentyl]methyl}-$N^4$-methyl-6'-(trifluoromethyl)[2,4'-bipyridine]-4,5,6-triamine | MS (ESI+) m/z 450.6 $(M+H)^+$ |
| 45 | 11 | Ethyl 1-(3-fluoro-4-formylphenyl)piperidine-4-carboxylate | MS (ESI+) m/z 280.6 $(M+H)^+$ |
| 46 | 12 | Methyl {[1-(5-formylpyrazin-2-yl)piperidin-4-yl]oxy}acetate | MS (ESI+) m/z 280.1 $(M+H)^+$ |
| 47 | 12 | Ethyl 3-[(2S)-4-(5-formylpyrazin-2-yl)-2-(methoxymethyl)piperazin-1-yl] propanoate | MS (ESI+) m/z 337.2 $(M+H)^+$ |

[Table 9]

| REx | PREx | Compound Name | Data |
|---|---|---|---|
| 48 | 11 | Ethyl [4-(5-formylpyrazin-2-yl)piperazin-1 -yl] acetate | MS (ESI+) m/z 279.3 $(M+H)^+$ |
| 49 | 11 | Ethyl 4-fluoro-1-(5-formylpyrazin-2-yl)piperidine-4-carboxylate | MS (ESI+) m/z 282.1 $(M+H)^+$ |
| 50 | 15 | Ethyl 4-fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylate | MS (ESI+) m/z 674.8 $(M+H)^+$ |
| 51 | 17 | Ethyl 1-(5-{5-[6-ethoxy-5-(trifluoromethyl)pyridin-3-yl]-7-[{[1-(methoxymethyl) cyclobutyl]methyl}(methyl)amino]-1H-imidazo[4,5-b]pyridin-2-yl} pyrazin-2-yl)piperidine-4-carboxylate | MS (ESI+) m/z 883.9 $(M+H)^+$ |
| 52 | 17 | Ethyl 1-(4-{5-[6-ethoxy-5-(trifluoromethyl)pyridin-3-yl]-7-[{[1-(methoxymethyl) cyclopentyl]methyl}(methyl)amino]-1H-imidazo[4,5-b]pyridin-2-yl}-3-fluorophenyl)piperidine-4-carboxylate | MS (ESI+) m/z 714.0 $(M+H)^+$ |

[Table 10]

| Ex | Data |
|---|---|
| 1 | MS (ESI+) m/z 643.8 $(M+H)^+$ |
| 2 | MS (ESI+) m/z 646.7 $(M+H)^+$ |
| 3 | MS (ESI+) m/z 655.8 $(M+H)^+$ |
| 4 | MS (ESI+) m/z 655.9 $(M+H)^+$<br>Elemental analysis value as $C_{32}H_{39}Cl_2F_3N_8O_4 + 0.8H_2O$<br>Calculated (%) C: 51.80 H: 5.52 N: 15.10<br>Found (%) C: 51.83 H: 5.50 N: 14.90 |
| 5 | MS (ESI+) m/z 695.9 $(M+H)^+$ |
| 6 | MS (ESI+) m/z 685.9 $(M+H)^+$ |
| 7 | MS (ESI+) m/z 643.9 $(M+H)^+$ |
| 8 | MS (ESI+) m/z 679.4 $(M+H)^+$ |
| 9 | MS (ESI+) m/z 669.9 $(M+H)^+$ |
| 10 | MS (ESI+) m/z 708.9 $(M+H)^+$ |
| 11 | MS (ESI+) m/z 712.1 $(M+H)^+$ |
| 12 | MS (ESI+) m/z 752.8 $(M+H)^+$ |
| 13 | MS (ESI+) m/z 722.9 $(M+H)^+$<br>Elemental analysis value as $C_{37}H_{45}F_3N_9NaO_3 + 3H_2O$<br>Calculated (%) C: 55.70 H: 6.44 N: 15.80<br>Found (%) C: 55.92 H: 6.82 N: 15.53 |
| 14 | MS (ESI+) m/z 723.0 $(M+H)^+$<br>Elemental analysis value as $C_{37}H_{45}F_3N_9NaO_3 + 3H_2O$<br>Calculated (%) C: 55.70 H: 6.44 N: 15.80<br>Found (%) C: 55.33 H: 6.29 N: 15.65 |
| 15 | MS (ESI+) m/z 709.0 $(M+H)^+$<br>Elemental analysis value as $C_{36}H_{43}F_3N_9NaO_3 + 3.9H_2O$<br>Calculated (%) C: 54.05 H: 6.40 N: 15.76<br>Found (%) C: 53.95 H: 6.15 N: 15.54 |

Pharmacological test (biological test) examples of the compounds used in the present invention are described below.

The pharmacological activity of the compound of each Example was examined by the following tests. In the following description, the compound of each Example is sometimes referred to as "test compound".

<Test Example 1: Evaluation of M3 PAM activity>

CHO-K1 cells in which human muscarinic M3 receptor gene (GenBank registration number: NM_000740.2) was introduced and M3 receptors were stably expressed (hereinafter, sometimes referred to as "M3R-expressing cells") were subcultured under the conditions of 37°C, 5% $CO_2$ using a growth medium. As the growth medium, alpha Modified Eagle Minimum Essential Medium (a-MEM, D8042, manufactured by Sigma) containing inactivated fetal bovine serum (Cat. No. 172012, manufactured by Sigma) having a final concentration of 10%, GlutaMAX (registered trademark) (Cat. No. 35050, manufactured by GIBCO) having a final concentration of 2 mM, penicillin having a final concentration of 20 U/mL and 20 μg/mL streptomycin (penicillin-streptomycin mixed solution, Cat. No. 26253-84, manufactured by NACALAI TESQUE, INC.), and G418 (Cat. No. 16513-26, manufactured by NACALAI TESQUE, INC.) having a final concentration of 0.2 mg/mL, was used.

On the day before the measurement of intracellular $Ca^{2+}$ concentration, the M3R-expressing cells were suspended in the growth medium and seeded at 40,000 cells/well on a 96-well plate with a black transparent bottom (Cat. No. 215006, manufactured by Porvair Sciences). The M3R-expressing cells seeded on the 96-well plate were cultured overnight under the conditions of 37°C, 5% $CO_2$.

Using a calcium measurement assay kit (Screen QuestFluo-8 Medium Removal Calcium Assay Kit, Cat. No. 36309, manufactured by AAT Bioquest), the $Ca^{2+}$ concentration in the M3R-expressing cells was measured according to the attached instructions. On the day of measurement, the growth medium was removed, a loading buffer was added to the 96-well plate in an amount of 100 μL/well, the cells were cultured under the conditions of 37°C, 5% $CO_2$ for 30 minutes, and then the plate was allowed to stand at room temperature for 30 minutes. This way, the M3R-expressing cells were loaded with a visible light-excited calcium indicator (Fluoro-8 (registered trademark), manufactured by AAT Bioquest). As the loading buffer, a buffer containing the calcium indicator was used. As the buffer, a Hanks' balanced salt solution (HBSS buffer) with pH 7.4 containing HEPES (Cat. No. 340-01371, manufactured by DOJINDO LABORATORIES) having a final concentration of 20 mM and probenecid (165-15472, manufactured by FUJIFILM Wako Pure Chemical Industries, Ltd.) having a final concentration of 2.5 mM was used. The Hanks' balanced salt solution was prepared by diluting 10 × HBSS (Cat. No. 14065-056, manufactured by GIBCO) 10-fold with ultrapure water.

Then, the 96-well plate was transferred into a fluorescence screening system (FLIPR TETRA (registered trademark), manufactured by Molecular Devices), and the intracellular $Ca^{2+}$ concentration-dependent fluorescence intensity by a test compound was measured. In the measurement of the fluorescence intensity, the excitation wavelength was set to 470 to 495 nm, and the fluorescence wavelength was set to 515 to 575 nm.

A vehicle containing the test compound or a vehicle alone was added to the 96-well plate, and the fluorescence intensity was measured for 2 minutes. HBSS buffer was used as the vehicle. The test compound was dissolved in dimethyl sulfoxide and then added to the HBSS buffer. At this time, the final concentration of dimethyl sulfoxide was set to 2.5%. In addition, the final concentration of the test compound was varied in the range of 0 to 30 μM. Then, acetylcholine with $EC_{20}$ (20% Effective Concentration), which gives an action of about 20% of the maximum activity, was added, and the fluorescence intensity was measured for 1 minute. At this time, $EC_{20}$ was in the range of about 10 to 30 nM.

A fluorescence intensity Lb when the HBSS buffer alone was added instead of the test compound, and acetylcholine having a final concentration of 100 μM was added, was defined as 100%, and a fluorescence intensity La when the HBSS buffer alone was added instead of the test compound, in the presence of acetylcholine with $EC_{20}$, was defined as 0%. In addition, the fluorescence intensity when the test compound was added was denoted by Lc, and an enhancement ratio Gr (unit: %) of the fluorescence intensity by the test compound was calculated according to the following equation (1). The M3 PAM activity of the test compound was evaluated based on the enhancement ratio Gr.

$$Gr = 100 \times (Lc - La)/(Lb - La) \qquad (1)$$

On the basis of the enhancement ratio Gr at each concentration of the test compound, $EC_{50}$ (50% Effective Concentration) for the enhancement ratio Gr was estimated from a logistic formula using a statistical program (SAS system, SAS Institute Japan). The results of this test are shown in Table 11. It was determined that the lower the $EC_{50}$ for the enhancement ratio Gr, the higher the M3 PAM activity.

[Table 11]

| Example | $EC_{50}$ (nM) |
|---|---|
| 1 | 0.930 |
| 2 | 0.690 |
| 3 | 0.949 |
| 4 | 2.66 |
| 5 | 0.288 |
| 6 | 1.96 |
| 7 | 2.14 |
| 8 | 3.04 |
| 9 | 2.59 |
| 10 | 1.40 |
| 11 | 4.58 |
| 12 | 4.92 |
| 13 | 2.17 |
| 14 | 2.07 |
| 15 | 2.33 |

As shown in Table 11, it was found that all the test compounds (compounds of Examples 1 to 15) exhibit high M3 PAM activity.

The fluorescence intensity did not increase when the test compound was added alone in the absence of acetylcholine. From this, it was found that the test compounds do not exhibit M3 receptor agonist activity.

As described above, it was confirmed that the test compounds have M3 PAM activity in vitro.

<Test Example 2: Evaluation of test compounds in Magnus test>

9- to 15-week-aged SD female rats (Japan SLC, Inc.) were exsanguinated to death by amputation of carotid artery under isoflurane anesthesia. Then, the ileum was removed from each rat, and cut to a length of 2 cm while excising the mesentery and connective tissue, to prepare an intestinal sample. In a Magnus tank, this sample was immersed and suspended in 20 mL of a Krebs solution at 37°C (118 mM NaCl, 4.7 mM KCl, 2.5 mM $CaCl_2$, 1.2 mM $MgSO_4$, 1.2 mM $KH_2PO_4$, 11 mM D-glucose, 20 mM $NaHCO_3$) aerated with a mixed gas of 95% $O_2$ and 5% $CO_2$. Then, a load of 1 g was applied to the sample to stabilize the tension.

One end of the sample was connected to an isotonic transducer (IT-10, Medical Agent), and tension (contraction force) data was imported to a personal computer via Power Lab (registered trademark) (A&D Instruments Limited). Carbachol (Carbamylcholine Chloride, Cat. No. 036-09841, manufactured by FUJIFILM Wako Pure Chemical Industries, Ltd.) was added to the Krebs solution in the Magnus tank such that the final concentration thereof was 1000 nM, and contraction of the sample was checked. A contraction force Tb of the sample at this time was defined as 100%. Then, the Magnus tank was washed with a Krebs solution three times to wash away carbachol, and the Magnus tank was finally filled with 20 mL of a Krebs solution.

Next, dimethyl sulfoxide (DMSO, Cat. No. 13445-74, manufactured by NACALAI TESQUE, INC.) was added to the Krebs solution in the Magnus tank, then carbachol was added to the Krebs solution in the Magnus tank such that the final concentration thereof was 50 nM, and contraction of the sample was checked. A contraction force Ta of the sample at this time was defined as 0%. Then, the Magnus tank was washed with a Krebs solution three times to wash away carbachol and DMSO, and the Magnus tank was finally filled with 20 mL of a Krebs solution. Next, a test compound dissolved in DMSO was added to the Krebs solution in the Magnus tank such that the final concentration thereof was 10 μM, then carbachol was added to the Krebs solution in the Magnus tank such that the final concentration thereof was 50 nM, and contraction of the sample was checked.

As described above, the contraction force Tb of the sample when carbachol was added at a final concentration of 1000 nM was defined as 100%, and the contraction force Ta of the sample when carbachol was added at a final

concentration of 50 nM under the condition that DMSO was added first instead of the test compound was defined as 0%. The contraction force of the sample when carbachol was added at a final concentration of 50 nM under the condition that the test compound was added first at a final concentration of 10 μM was denoted by Tc, and an enhancement ratio P (unit: %) of the contraction force by the test compound was calculated according to the following equation (2). The increase in contraction force by the test compound was evaluated on the basis of the enhancement ratio P. The number of cases in each addition group was 3 to 7.

$$P = 100 \times (Tc - Ta)/(Tb - Ta) \qquad (2)$$

[Table 12]

| Example | Enhancement ratio (%) |
|---|---|
| 1 | 30.39 ± 8.34 |
| 2 | 61.50 ± 8.00 |
| 3 | 57.33 ± 7.24 |
| 4 | 59.29 ± 7.17 |
| 5 | 33.52 ± 3.39 |
| 6 | 26.70 ± 5.04 |
| 7 | 25.76 ± 6.38 |
| 8 | 12.40 ± 5.31 |
| 9 | 36.86 ± 6.43 |
| 10 | 19.27 ± 5.52 |
| 11 | 30.05 ± 3.79 |
| 12 | 49.28 ± 11.01 |
| 13 | 17.71 ± 12.74 |
| 14 | 65.06 ± 14.97 |
| 15 | 79.35 ± 12.20 |

The results of the Magnus test are shown in Table 12. In Table 12, the enhancement ratio P is shown as an average value ± standard error. All the test compounds increased the enhancement ratio P. Accordingly, it was found that the test compounds are effective for enhancing gastrointestinal function.

Furthermore, each test compound does not exhibit agonist activity against M3 receptors when used alone, but has an ileum contraction effect in the presence of carbachol. Accordingly, the test compounds having M3 PAM activity can enhance the signal levels of M3 receptors under more physiological conditions, and are expected to be therapeutically promising for diseases involving M3 receptors (especially, gastrointestinal diseases). In addition, the test compounds may avoid a cholinergic side effect (cholinergic crisis) which has been reported on existing pharmaceutical drugs (for example, distigmine bromide), and thus, the compounds may be therapeutic drugs having more excellent safety.

<Test Example 3: Evaluation of test compounds in mouse constipation model>

Loperamide, a μ-opioid receptor agonist, suppresses contraction of the intestinal tract and causes a delay in intestinal transport capacity. For this reason, mice administered with loperamide are known as an experimental model of constipation (hereinafter, sometimes referred to as "constipation model mice") (Acta gastroenterologica latinoamericana, 1991, Vol. 21, No. 1, p. 3-9.). Therefore, the effectiveness, in constipation model mice, of the test compounds exhibiting M3 PAM activity was examined.

7-week-aged male ICR mice (Japan SLC, Inc.) were raised and tamed in a wire mesh cage having a width of 20 cm, a depth of 23 cm, and a height of 15 cm for 1 week or longer from the time of arrival of the mice. Then, loperamide (Loperamide Hydrochloride, Cat. No. 129-05721, manufactured by FUJIFILM Wako Pure Chemical Industries, Ltd.) was

orally administered at 5 mg/kg. After 20 minutes from the administration of loperamide, a test compound or a vehicle (0.5% methyl cellulose solution) was orally administered at 10 mg/kg, and the number of feces after 6 hours was counted. At this time, loperamide was dissolved in water (Otsuka distilled water, manufactured by Otsuka Pharmaceutical Factory, Inc.), the test compound was suspended in a 0.5% methyl cellulose solution, and the administration volume thereof was set to 10 mL/kg. In a loperamide non-administration group (Normal group), only water was orally administered at 10 mL/kg. The number of cases in each administration group was 5 to 6.

The results of the constipation model mice are shown in FIG. 1 to FIG. 8. FIG. 1 and FIG. 5 show the results when compounds of Examples 1 to 4 were administered, FIG. 2 and FIG. 6 show the results when compounds of Examples 6 and 7 were administered, FIG. 3 and FIG. 7 show the results when compounds of Examples 8 to 11 were administered, and FIG. 4 and FIG. 8 show the results when compounds of Examples 12 to 15 were administered. The vertical axis in FIG. 1 to FIG. 4 indicates the number of feces per mouse (unit: pieces), and the vertical axis in FIG. 5 to FIG. 8 indicates the total weight of feces per mouse after drying (unit: mg). In each figure, "N" and "Vh" indicate the Normal group and the Vehicle group, respectively. In addition, the numbers "1 to 4 and 6 to 15" in the figures indicate the compounds of Examples 1 to 4 and 6 to 15, respectively. Hereinafter, the total weight of feces after drying is sometimes referred to as "dry weight". In the group in which only loperamide and the vehicle were administered (Vehicle group), the number of feces and the dry weight decreased as compared to those in the loperamide non-administration group (Normal group). From this, establishment of a constipation model was confirmed.

In each test compound administration group, the number of feces and the dry weight increased with respect to the Vehicle group, and constipation was improved. In addition, in each test compound administration group, both the number of feces and the dry weight increased with respect to the Vehicle group. Therefore, it was confirmed that the administration of each test compound did not merely cause a large number of small feces to be discharged and increase only the number of times of defecation, but also increased the amount of feces discharged.

From the above results, it was found that the test compounds exhibit effectiveness for irritable bowel syndrome, functional constipation, and opioid-induced constipation. It was visually confirmed that no diarrhea was observed in any of the Normal group, the Vehicle group, and the test compound administration groups.

<Test Example 4: Experiment of measurement of secreted saliva amount in anesthetized mice>

8-week-aged male ICR mice (Japan SLC, Inc.) were anesthetized with urethane (1650 mg/kg, intraperitoneal administration). After about 1 hour, a test compound or a vehicle (physiological saline containing 10% DMSO (OTSUKA NORMAL SALINE, manufactured by Otsuka Pharmaceutical Factory, Inc.)) was intravenously administered at 10 mg/kg, and immediately after that, one or two absorbent cotton wool balls (0.1-0.2 mg) (Roller Cotton (cotton ball) SS, manufactured by Nichiei Co., Ltd.) were inserted into the mouth. The difference between the weight of the absorbent cotton balls after 30 minutes from insertion and the weight of the absorbent cotton balls before insertion was defined as an amount of saliva secreted. At this time, the test compound was dissolved in DMSO and then diluted 10-fold with physiological saline. The administration volumes of the test compound and the vehicle were each set to 5 mL/kg. The experiment was carried out in three parts. The number of cases in each administration group was 3-4.

[Table 13]

| | Example | Amount of saliva secreted (mg) |
|---|---|---|
| Experiment #1 | Vehicle | 1.23±0.80 |
| | 1 | 9.63±2.90 |
| | 2 | 9.73±3.61 |
| | 3 | 27.23±5.94 |
| | 4 | 77.35±16.66 |
| | 5 | 66.30±15.32 |
| Experiment #2 | Vehicle | 1.73±1.68 |
| | 6 | 11.37±2.87 |
| | 7 | 73.40±28.54 |
| | 8 | 49.13±11.95 |
| | 9 | 34.73±11.39 |
| | 10 | 329.83±49.36 |

(continued)

| | Example | Amount of saliva secreted (mg) |
|---|---|---|
| Experiment #3 | Vehicle | 0.63±0.33 |
| | 11 | 51.85±7.37 |
| | 12 | 131.75±28.63 |
| | 13 | 214.40±51.06 |
| | 14 | 142.70±23.51 |
| | 15 | 304.98±58.25 |

The results of the test of secreted saliva amount measurement are shown in Table 13. In Table 13, the amount of saliva secreted (unit: mg) is shown as an average value ± standard error. The compounds of Examples 1 to 5 were evaluated in Experiment #1, the compounds of Examples 6 to 10 were evaluated in Experiment #2, and the compounds of Examples 11 to 15 were evaluated in Experiment #3. In each test compound administration group, the amount of saliva secreted increased as compared to that in the Vehicle group (only vehicle was administered). In the Vehicle group and each test compound administration group, no aspiration of saliva into the trachea was observed. In this test, pilocarpine (a muscarinic receptor agonist similar to carbachol) also exhibited a salivation promoting effect.

As shown in Test Examples 1 to 4 described above, the compound of the present invention exhibits M3 PAM activity and also exhibits effectiveness in the in vivo model, and is useful as a therapeutic agent or a prophylactic agent for functional gastrointestinal disorders or xerostomia.

INDUSTRIAL APPLICABILITY

The present invention can be used for therapeutic agents and prophylactic agents for functional gastrointestinal disorders and xerostomia.

## Claims

1. A therapeutic agent or prophylactic agent for a functional gastrointestinal disorder, comprising an azabenzimidazole compound, or a pharmaceutically acceptable salt thereof, or a solvate thereof, as an active ingredient, the azabenzimidazole compound being a compound of the formula [1]:

[Chem. 1]

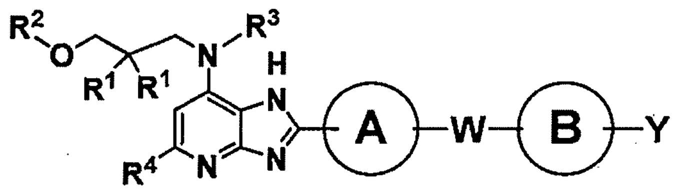

**[1]**

wherein:

$R^1$ is a hydrogen atom or alkyl, or the two $R^1$s combine with the adjacent carbon atom to form a 3- to 7-membered cycloalkyl or an oxygen-containing non-aromatic heterocycle;
$R^2$ is a hydrogen atom, alkyl, cycloalkyl, alkyl substituted with cycloalkyl, or alkoxyalkyl;
$R^3$ is a hydrogen atom, alkyl, or alkoxyalkyl;
$R^4$ is pyridyl optionally substituted with one or two groups selected from the group consisting of alkyl, trihaloalkyl, alkoxy, cyano, and cycloalkyl, or phenyl optionally substituted with 1 to 3 groups selected from the group consisting of trihaloalkyl, halogen, alkoxy, and cycloalkyl;

A is a group of the formula A-1, A-2, A-3, A-4, or A-5:

[Chem. 2]

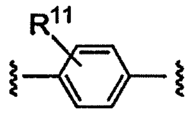
A-1

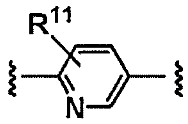
A-2

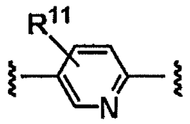
A-3

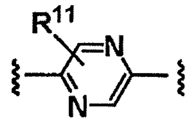
A-4

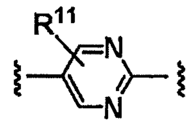
A-5

wherein the bond on the left side of each group is attached to the 2-position of the azabenzimidazole in the formula [1], the bond on the right side is attached to W in the formula [1], and $R^{11}$ is a group selected from a hydrogen atom, halogen, alkyl, alkoxy, and nitro; W is a bond or a group of the formula W-1, W-2, or W-3:

[Chem. 3]

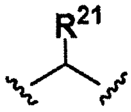
W-1

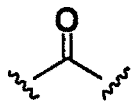
W-2

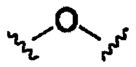
W-3

wherein $R^{21}$ is a hydrogen atom or alkyl;
B is a group of the formula B-1, B-2, B-3, or B-4:

[Chem. 4]

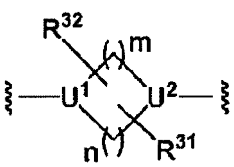
B-1

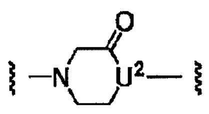
B-2

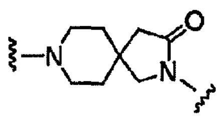
B-3

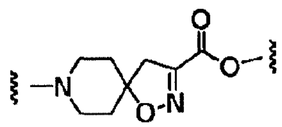
B-4

wherein the bond on the left side of each group is attached to W in the formula [1], the bond on the right side is attached to Y in the formula [1], $U^1$ is a nitrogen atom or $CR^{41}$, $U^2$ is a nitrogen atom or $CR^{42}$, $R^{41}$ and $R^{42}$ are each independently a hydrogen atom, alkyl, halogen, or a hydroxyl group, m and n are each 1, 2, or 3, and $R^{31}$ and $R^{32}$ are each independently a hydrogen atom, alkyl, halogen, or alkoxyalkyl, or $R^{31}$ and $R^{32}$ combine with an adjacent carbon atom to form an alkylene bridge, provided that $R^{31}$ and $R^{32}$ substitute at any substitutable positions other than $U^1$ and $U^2$; and
Y is a hydrogen atom or a group of any one of the formulae Y-1 to Y-4, Y-11 to Y-16:

[Chem. 5]

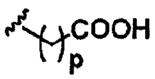
Y-1

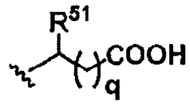
Y-2

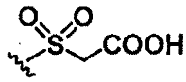
Y-3

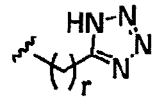
Y-4

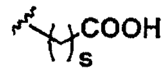
Y-11

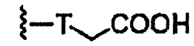
Y-12

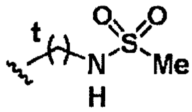
Y-13

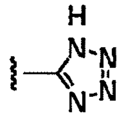
Y-14

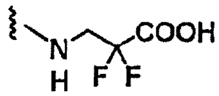
Y-15

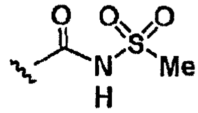
Y-16

wherein $R^{51}$ is alkyl, p is 1, 2, or 3, q is 0, 1, or 2, r is 1, 2, or 3, T is O, S, $SO_2$, or $NR^{61}$ wherein $R^{61}$ is a hydrogen atom or alkyl, s is 0, 1, 2, or 3, and t is 0 or 1, with the proviso that one of the following cases (a) to (d) is selected:

(a) when W is a bond,

if B is B-1 or B-2 and $U^2$ is a nitrogen atom, then Y is Y-1, Y-2, Y-3, or Y-4,
if B is B-1 or B-2 and $U^2$ is $CR^{42}$ wherein $R^{42}$ is as defined above, then $U^1$ is a nitrogen atom and Y is Y-11, Y-12, Y-13, Y-14, Y-15, or Y-16, and
if B is B-3 or B-4, then Y is a hydrogen atom;

(b) when W is W-1,

if B is B-1, $U^1$ is a nitrogen atom, and $U^2$ is a nitrogen atom, then Y is Y-1, Y-2, Y-3, or Y-4, and
if B is B-1, $U^1$ is a nitrogen atom, and $U^2$ is $CR^{42}$ wherein $R^{42}$ is as defined above, then Y is Y-11, Y-12, Y-13, Y-14, Y-15, or Y-16;

(c) when W is W-2,

if B is B-1 or B-2, $U^1$ is a nitrogen atom, and $U^2$ is a nitrogen atom, then Y is Y-1, Y-2, Y-3, or Y-4,
if B is B-1 or B-2, $U^1$ is a nitrogen atom, and $U^2$ is $CR^{42}$ wherein $R^{42}$ is as defined above, then Y is Y-11, Y-12, Y-13, Y-14, Y-15, or Y-16, and
if B is B-3 or B-4, then Y is a hydrogen atom; and

(d) when W is W-3,
if B is B-1, $U^1$ is $CR^{41}$ wherein $R^{41}$ is as defined above, and $U^2$ is a nitrogen atom, then Y is Y-1, Y-2, Y-3, or Y-4.

**2.** The therapeutic agent or prophylactic agent according to claim 1, wherein W is a bond.

**3.** The therapeutic agent or prophylactic agent according to claim 1 or 2, wherein

(1) B is B-1 or B-2, $U^2$ is a nitrogen atom, and Y is Y-1, Y-2, or Y-3,
(2) B is B-1 or B-2, $U^2$ is $CR^{42}$, and Y is Y-11, Y-12, or Y-15, or
(3) B is B-4 and Y is a hydrogen atom.

**4.** The therapeutic agent or prophylactic agent according to claim 3, wherein $R^4$ is pyridyl substituted with trihaloalkyl and a group selected from the group consisting of alkyl, trihaloalkyl, alkoxy, cyano, and cycloalkyl.

**5.** The therapeutic agent or prophylactic agent according to claim 4, wherein A is A-4.

**6.** The therapeutic agent or prophylactic agent according to any one of claims 1 to 5, wherein the azabenzimidazole compound is any one of the following (1) to (15):

(1) [4-(5-{5-[3-fluoro-5-(trifluoromethyl)phenyl]-7-[{[1-(methoxymethyl)cyclobutyl]methyl)(methyl)amino]-1H-imidazo[4,5-b]pyridin-2-yl}pyrazin-2-yl)piperazin-1-yl]acetic acid,

(2) 4-fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,

(3) 1-(5-{5 - [6-ethoxy-5-(trifluoromethyl)pyridin-3-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,

(4) 1-(5-{5-[2-ethoxy-6-(trifluoromethyl)pyridin-4-yl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,

(5) {[1-(5-{5-[2-cyclopropyl-6-(trifluoromethyl)pyridin-4-yl]-7-[{[1-(methoxymethyl)cyclopentyl]methyl} (methyl)amino]-1H-imidazo[4,5-b]pyridin-2-yl}pyrazin-2-yl)piperidin-4-yl] oxy} acetic acid,

(6) 1-(4- {5-[6-ethoxy-5-(trifluoromethyl)pyridin-3-yl]-7-[{[1-(methoxymethyl)cyclopentyl]methyl} (methyl)amino]-1H-imidazo[4,5-b]pyridin-2-yl} -3 - fluorophenyl)piperidine-4-carboxylic acid,

(7) 1-(5-{5- [2-ethoxy-6-(trifluoromethyl)pyridin-4-yl] -7- [(3 -methoxy-2,2-dimethylpropyl)(methyl)amino] -1H-imidazo[4,5-b]pyridin-2-yl}pyrazin-2-yl)piperidine-4-carboxylic acid,

(8) 1-(5-{5-[6-cyclopropyl-5-(trifluoromethyl)pyridin-3 -yl]-7-[{[1-(ethoxymethyl)cyclopentyl]methyl}(methyl)amino]-1H-imidazo[4,5-b]pyridin-2-yl}pyrazin-2-yl)piperidine-4-carboxylic acid,

(9) 1-(5-{5-[2-ethoxy-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidine-4-carboxylic acid,

(10) 3-[4-(5-{5-[6-cyclopropyl-5-(trifluoromethyl)pyridin-3-yl]-7-[{[1-(methoxymethyl)cyclohexyl]methyl} (methyl)amino]-1H-imidazo[4,5-b]pyridin-2-yl}pyrazin-2-yl)piperazin-1-yl]propanoic acid,

(11) [4-(4-{5-[6-cyclopropyl-5-(trifluoromethyl)pyridin-3-yl]-7-[{[1-(methoxymethyl)cyclopentyl]methyl} (methyl)amino]-1H-imidazo[4,5-b]pyridin-2-yl}-3-fluorophenoxy)piperidin-1-yl]acetic acid,

(12) 3-[(2S)-4-(5-{5-[6-cyclopropyl-5-(trifluoromethyl)pyridin-3-yl]-7-[({[1-(methoxymethyl)cyclohexyl]methyl}(methyl)amino]-1H-imidazo[4,5-b]pyridin-2-yl}pyrazin-2-yl)-2-(methoxymethyl)piperazin-1-yl]propanoic acid,

(13) 3-[(3R)-4-(5-15-[6-cyclopropyl-5-(trifluoromethyl)pyridin-3-yl]-7-[{[1-(ethoxymethyl)cyclopentyl]methyl} (methyl)amino]-1H-imidazo[4,5-b]pyridin-2-yl}pyrazin-2-yl)-3-methylpiperazin-1-yl]propanoic acid,

(14) 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid, and

(15) 3-[(3R)-4-(5-{5-[6-cyclopropyl-5-(trifluoromethyl)pyridin-3-yl]-7-[{[1-(methoxymethyl)cyclopentyl]methyl} (methyl)amino]-1H-imidazo[4,5-b]pyridin-2-yl}pyrazin-2-yl)-3-methylpiperazin-1-yl]propanoic acid.

**7.** The therapeutic agent or prophylactic agent according to any one of claims 1 to 6, wherein the functional gastrointestinal disorder is irritable bowel syndrome (IBS).

**8.** The therapeutic agent or prophylactic agent according to any one of claims 1 to 6, wherein the functional gastrointestinal disorder is functional constipation.

**9.** A therapeutic agent or prophylactic agent for xerostomia, comprising an azabenzimidazole compound, or a pharmaceutically acceptable salt thereof, or a solvate thereof, as an active ingredient, the azabenzimidazole compound being a compound of the formula [1]:

[Chem. 6]

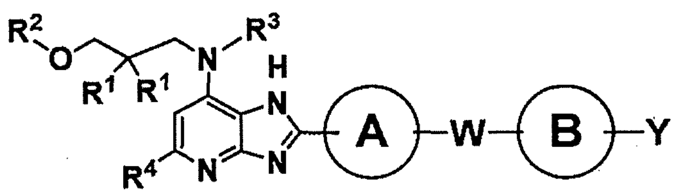

**[1]**

wherein:

$R^1$ is a hydrogen atom or alkyl, or the two $R^1$s combine with the adjacent carbon atom to form a 3- to 7-membered cycloalkyl or an oxygen-containing non-aromatic heterocycle;
$R^2$ is a hydrogen atom, alkyl, cycloalkyl, alkyl substituted with cycloalkyl, or alkoxyalkyl;
$R^3$ is a hydrogen atom, alkyl, or alkoxyalkyl;
$R^4$ is pyridyl optionally substituted with one or two groups selected from the group consisting of alkyl, trihaloalkyl, alkoxy, cyano, and cycloalkyl, or phenyl optionally substituted with 1 to 3 groups selected from the group consisting of trihaloalkyl, halogen, alkoxy, and cycloalkyl;
A is a group of the formula A-1, A-2, A-3, A-4, or A-5:

[Chem. 7]

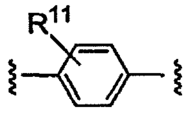
A-1

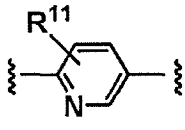
A-2

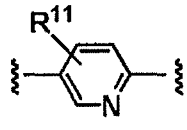
A-3

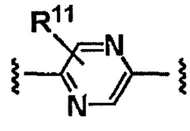
A-4

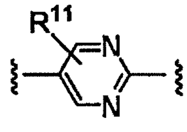
A-5

wherein the bond on the left side of each group is attached to the 2-position of the azabenzimidazole in the formula [1], the bond on the right side is attached to W in the formula [1], and $R^{11}$ is a group selected from a hydrogen atom, halogen, alkyl, alkoxy, and nitro; W is a bond or a group of the formula W-1, W-2, or W-3:

[Chem. 8]

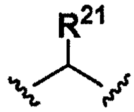
W-1

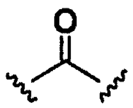
W-2

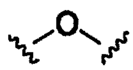
W-3

wherein $R^{21}$ is a hydrogen atom or alkyl;
B is a group of the formula B-1, B-2, B-3, or B-4:

[Chem. 9]

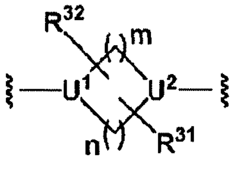
B-1

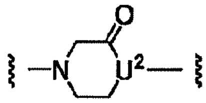
B-2

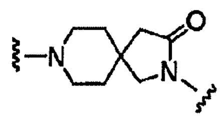
B-3

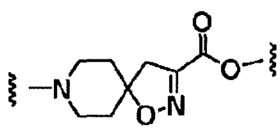
B-4

wherein the bond on the left side of each group is attached to W in the formula [1], the bond on the right side is attached to Y in the formula [1], $U^1$ is a nitrogen atom or $CR^{41}$, $U^2$ is a nitrogen atom or $CR^{42}$, $R^{41}$ and $R^{42}$ are each independently a hydrogen atom, alkyl, halogen, or a hydroxyl group, m and n are each 1, 2, or 3, and $R^{31}$ and $R^{32}$ are each independently a hydrogen atom, alkyl, halogen, or alkoxyalkyl, or $R^{31}$ and $R^{32}$ combine with an adjacent carbon atom to form an alkylene bridge, provided that $R^{31}$ and $R^{32}$ substitute at any substitutable positions other than $U^1$ and $U^2$; and

Y is a hydrogen atom or a group of any one of the formulae Y-1 to Y-4, Y-11 to Y-16:

[Chem. 10]

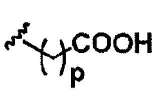

Y-1

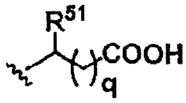

Y-2

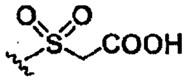

Y-3

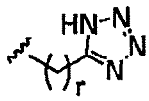

Y-4

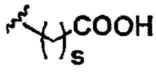

Y-11

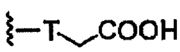

Y-12

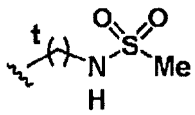

Y-13

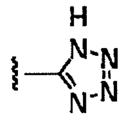

Y-14

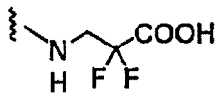

Y-15

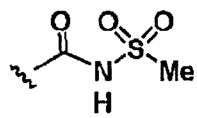

Y-16

wherein $R^{51}$ is alkyl, p is 1, 2, or 3, q is 0, 1, or 2, r is 1, 2, or 3, T is O, S, $SO_2$, or $NR^{61}$ wherein $R^{61}$ is a hydrogen atom or alkyl, s is 0, 1, 2, or 3, and t is 0 or 1, with the proviso that one of the following cases (a) to (d) is selected:

(a) when W is a bond,

if B is B-1 or B-2 and $U^2$ is a nitrogen atom, then Y is Y-1, Y-2, Y-3, or Y-4,
if B is B-1 or B-2 and $U^2$ is $CR^{42}$ wherein $R^{42}$ is as defined above, then $U^1$ is a nitrogen atom and Y is Y-11, Y-12, Y-13, Y-14, Y-15, or Y-16, and
if B is B-3 or B-4, then Y is a hydrogen atom;

(b) when W is W-1,

if B is B-1, $U^1$ is a nitrogen atom, and $U^2$ is a nitrogen atom, then Y is Y-1, Y-2, Y-3, or Y-4, and
if B is B-1, $U^1$ is a nitrogen atom, and $U^2$ is $CR^{42}$ wherein $R^{42}$ is as defined above, then Y is Y-11, Y-12, Y-13, Y-14, Y-15, or Y-16;

(c) when W is W-2,

if B is B-1 or B-2, $U^1$ is a nitrogen atom, and $U^2$ is a nitrogen atom, then Y is Y-1, Y-2, Y-3, or Y-4,
if B is B-1 or B-2, $U^1$ is a nitrogen atom, and $U^2$ is $CR^{42}$ wherein $R^{42}$ is as defined above, then Y is Y-11, Y-12, Y-13, Y-14, Y-15, or Y-16, and
if B is B-3 or B-4, then Y is a hydrogen atom; and

(d) when W is W-3,
if B is B-1, $U^1$ is $CR^{41}$ wherein $R^{41}$ is as defined above, and $U^2$ is a nitrogen atom, then Y is Y-1, Y-2, Y-3, or Y-4.

**10.** The therapeutic agent or prophylactic agent according to claim 9, wherein W is a bond.

**11.** The therapeutic agent or prophylactic agent according to claim 9 or 10, wherein

(1) B is B-1 or B-2, $U^2$ is a nitrogen atom, and Y is Y-1, Y-2, or Y-3,
(2) B is B-1 or B-2, $U^2$ is $CR^{42}$, and Y is Y-11, Y-12, or Y-15, or
(3) B is B-4 and Y is a hydrogen atom.

**12.** The therapeutic agent or prophylactic agent according to claim 11, wherein $R^4$ is pyridyl substituted with trihaloalkyl and a group selected from the group consisting of alkyl, trihaloalkyl, alkoxy, cyano, and cycloalkyl.

**13.** The therapeutic agent or prophylactic agent according to claim 12, wherein A is A-4.

**14.** The therapeutic agent or prophylactic agent according to any one of claims 9 to 13, wherein the azabenzimidazole compound is any one of the following (1) to (15):

(1) [4-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperazin-1-yl]acetic acid,
(2) 4-fluoro-1-(5-{5-[3-fluoro-5-(trifluoromethyl)phenyl]-7-[{[1-(methoxymethyl)cyclobutyl]methyl}(methyl)amino]-1H-imidazo[4,5-b]pyridin-2-yl}pyrazin-2-yl)piperidine-4-carboxylic acid,
(3) 1-(5-{5-[6-ethoxy-5-(trifluoromethyl)pyridin-3-yl]-7-[{[1-(methoxymethyl)cyclobutyl]methyl} (methyl)amino]-1H-imidazo[4,5-b]pyridin-2-yl} pyrazin-2-yl)piperidine-4-carboxylic acid,
(4) 1-(5-{5-[2-ethoxy-6-(trifluoromethyl)pyridin-4-yl]-7-[{[1-(methoxymethyl)cyclobutyl]methyl}(methyl)amino] -1H-imidazo[4,5-b]pyridin-2-yl} pyrazin-2-yl)piperidine-4-carboxylic acid,
(5) {[1 -(5-{5-[2-cyclopropyl-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidin-4-yl]oxy}acetic acid,
(6) 1-(4- {5 - [6-ethoxy-5-(trifluoromethyl)pyridin-3 -yl] -7-[{[1-(methoxymethyl)cyclopentyl]methyl}(methyl)amino]-1H-imidazo[4,5-b]pyridin-2-yl}-3-fluorophenyl)piperidine-4-carboxylic acid,
(7) 1-(5-{5-[2-ethoxy-6-(trifluoromethyl)pyridin-4-yl]-7-[(3-methoxy-2,2-dimethylpropyl)(methyl)amino]-1H-imidazo[4,5-b]pyridin-2-yl}pyrazin-2-yl)piperidine-4-carboxylic acid,
(8) 1-(5-{5-[6-cyclopropyl-5-(trifluoromethyl)pyridin-3-yl]-7-[{[1-(ethoxymethyl)cyclopentyl]methyl}(methyl)amino]-1H-imidazo[4,5-b]pyridin-2-yl}pyrazin-2-yl)piperidine-4-carboxylic acid,
(9) 1-(5-{5-[2-ethoxy-6-(trifluoromethyl)pyridin-4-yl]-7-[{[1-(methoxymethyl)cyclopentyl]methyl}(methyl)amino]-1H-imidazo[4,5-b]pyridin-2-yl}pyrazin-2-yl)piperidine-4-carboxylic acid,
(10) 3-[4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)piperazin-1-yl]propanoic acid,
(11) [4-(4-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}-3-fluorophenoxy)piperidin-1-yl] acetic acid,
(12) 3-[(2S)-4-(5-{5-[6-cyclopropyl-5-(trifluoromethyl)pyridin-3-yl]-7-[{[1-(methoxymethyl)cyclohexyl]methyl}(methyl)amino]-1H-imidazo[4,5-b]pyridin-2-yl}pyrazin-2-yl)-2-(methoxymethyl)piperazin-1-yl]propanoic acid,
(13) 3-[(3R)-4-(5-{5-[6-cyclopropyl-5-(trifluoromethyl)pyridin-3-yl]-7-[{[1-(ethoxymethyl)cyclopentyl]methyl}(methyl)amino] -1H-imidazo[4,5-b]pyridin-2-yl}pyrazin-2-yl)-3-methylpiperazin-1-yl]propanoic acid,
(14) 3-[(3R)-4-(5-{5-[2-cyclopropyl-6-(trifluoromethyl)pyridin-4-yl]-7-[{[1-(methoxymethyl)cyclohexyl]methyl}(methyl)amino]-1H-imidazo[4,5-b]pyridin-2-yl}pyrazin-2-yl)-3-methylpiperazin-1-yl]propanoic acid, and
(15) 3-[(3R)-4-(5-{5-[6-cyclopropyl-5-(trifluoromethyl)pyridin-3-yl]-7-[{[1-(methoxymethyl)cyclopentyl]methyl}(methyl)amino]-1H-imidazo[4,5-b]pyridin-2-yl}pyrazin-2-yl)-3-methylpiperazin-1-yl]propanoic acid.

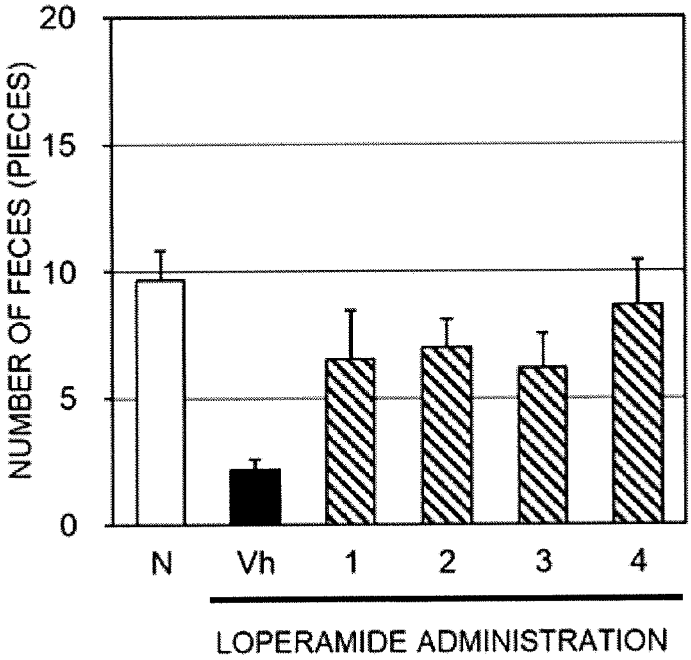
20
15
10
5
0
NUMBER OF FECES (PIECES)
N
Vh
1
2
3
4
LOPERAMIDE ADMINISTRATION

Fig.1

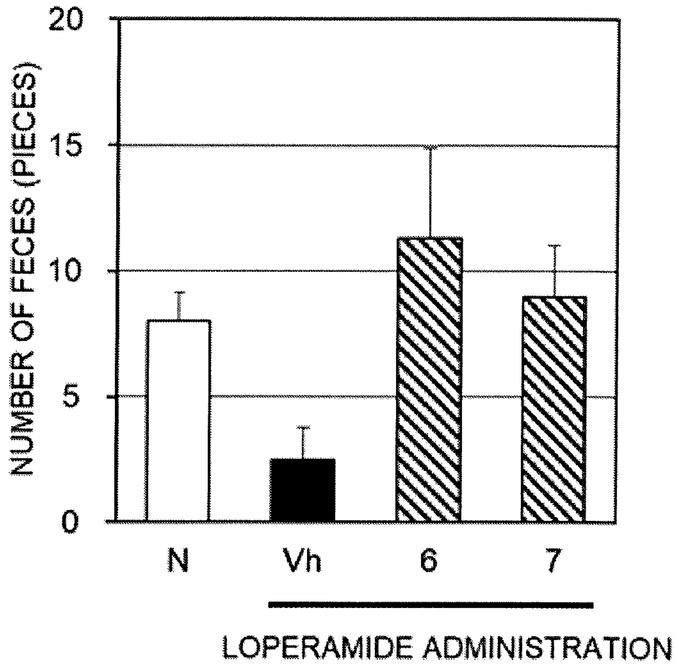
20
15
10
5
0
NUMBER OF FECES (PIECES)
N
Vh
6
7
LOPERAMIDE ADMINISTRATION

Fig.2

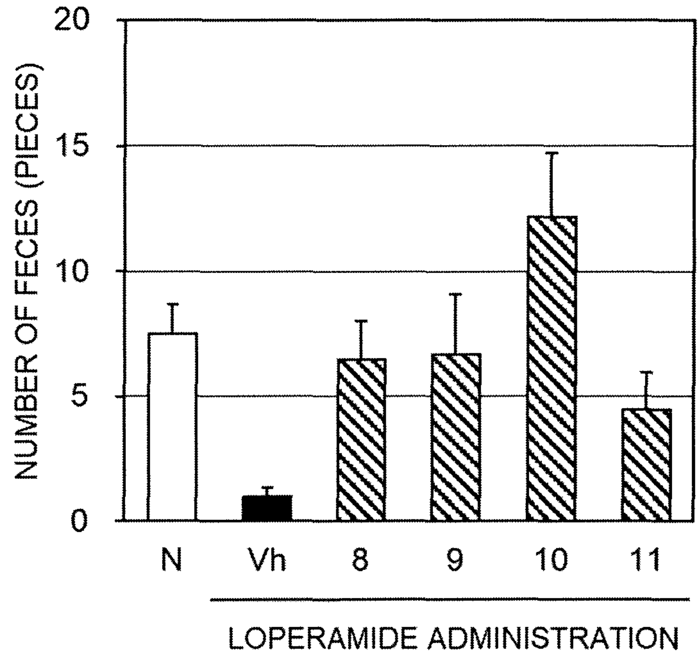
20
15
10
5
0
NUMBER OF FECES (PIECES)
N
Vh
8
9
10
11
LOPERAMIDE ADMINISTRATION

Fig.3

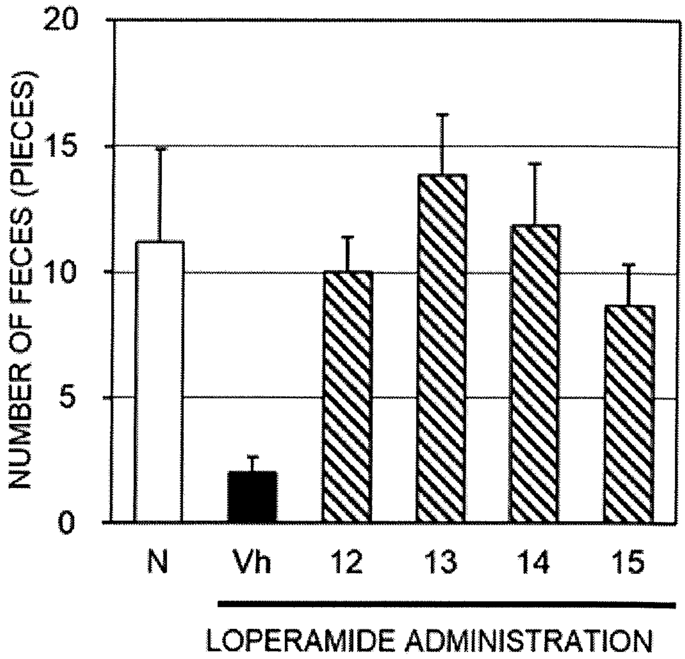

NUMBER OF FECES (PIECES)
20
15
10
5
0
N
Vh
12
13
14
15
LOPERAMIDE ADMINISTRATION


Fig.4

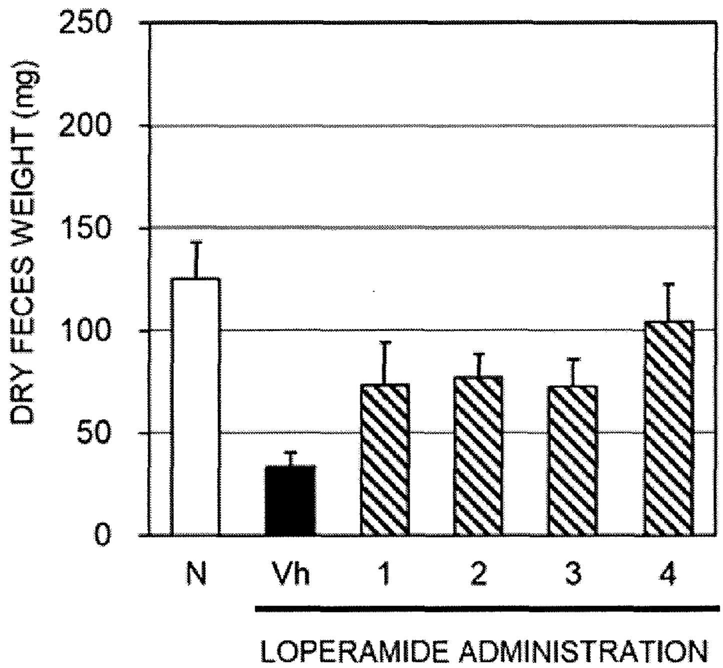
DRY FECES WEIGHT (mg)
250
200
150
100
50
0
N
Vh
1
2
3
4
LOPERAMIDE ADMINISTRATION

Fig.5

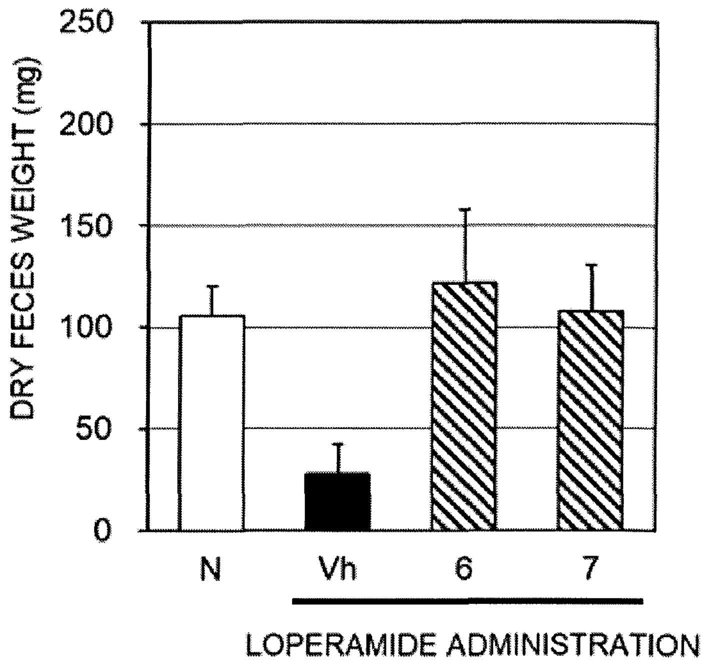
250
200
150
100
50
0
DRY FECES WEIGHT (mg)
N
Vh
6
7
LOPERAMIDE ADMINISTRATION

Fig.6

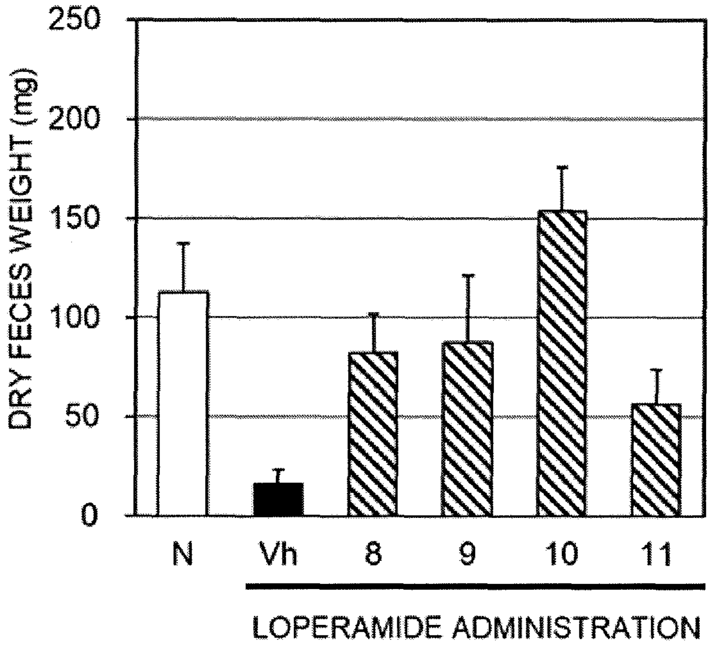
250
200
150
100
50
0
DRY FECES WEIGHT (mg)
N
Vh
8
9
10
11
LOPERAMIDE ADMINISTRATION

Fig.7

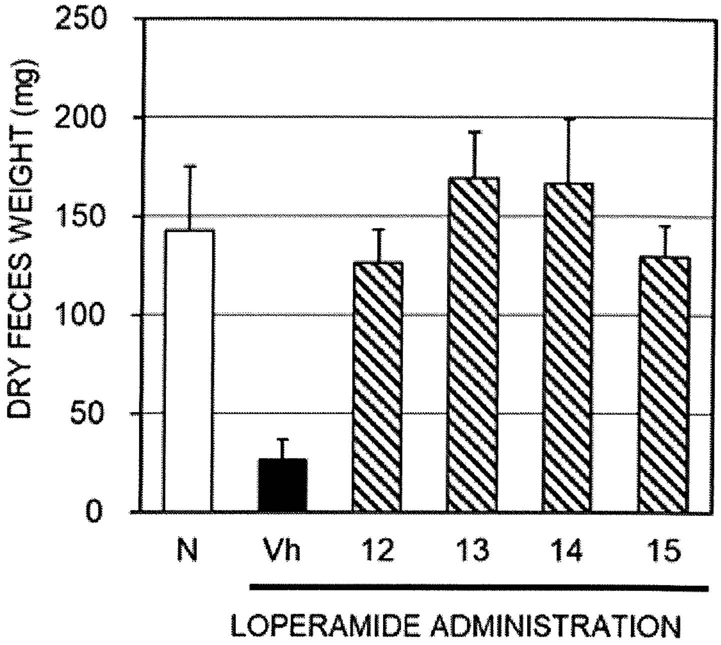
250
200
150
100
50
0
DRY FECES WEIGHT (mg)
N
Vh
12
13
14
15
LOPERAMIDE ADMINISTRATION

Fig.8

## INTERNATIONAL SEARCH REPORT

International application No. PCT/JP2020/042259

A. CLASSIFICATION OF SUBJECT MATTER
A61K 31/496(2006.01)i; A61K 31/4545(2006.01)i; A61K 31/497(2006.01)i; A61P 1/02(2006.01)i; A61P 1/04(2006.01)i; A61P 1/10(2006.01)i
FI: A61K31/496; A61K31/4545; A61K31/497; A61P1/02; A61P1/04; A61P1/10
According to International Patent Classification (IPC) or to both national classification and IPC

B. FIELDS SEARCHED

Minimum documentation searched (classification system followed by classification symbols)
A61K31/496; A61K31/4545; A61K31/497; A61P1/02; A61P1/04; A61P1/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | JP 2009-542802 A (DAEWOONG PHARMACEUTICAL CO., LTD.) 03 December 2009 (2009-12-03) in particular, claims 1, 4, 6 | 1-14 |
| A | WO 2005/115399 A2 (NEUROGEN CORPORATION) 08 December 2005 (2005-12-08) in particular, claims 1, 15, 35, 39 | 1-14 |
| A | WO 2011/018894 A1 (RAQUALIA PHARMA INC.) 17 February 2011 (2011-02-17) in particular, claims 1, 7, 10 | 1-14 |
| A | WO 2018/118736 A1 (MERCK SHARP & DOHME CORP.) 28 June 2018 (2018-06-28) in particular, page 2, line 30 to page 3, line 5, page 130, line 4 to page 132, line 1, claims 1-21 | 1-14 |
| P, X<br>P, A | WO 2019/216294 A1 (NIPPON SHINYAKU CO., LTD.) 14 November 2019 (2019-11-14) in particular, claims 1-11, paragraph [0293], examples 1-4, 6, 8, 10, 11, 14, 16, 20, 37, 38, 42, 68 | 9-14<br>1-8 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 December 2020 (28.12.2020) | 12 January 2021 (12.01.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/042259

| Patent Documents referred in the Report | Publication Date | Patent Family | Publication Date |
|---|---|---|---|
| JP 2009-542802 A | 03 Dec. 2009 | US 2009/0209540 A1 in particular, claims 1, 4, 6<br>WO 2008/007900 A1<br>EP 2054405 A1<br>CA 2656810 A1<br>CN 101490037 A<br>AU 2007273333 A1<br>NZ 574697 A<br>MY 157033 A<br>KR 10-2009-0112624 A | |
| WO 2005/115399 A2 | 08 Dec. 2005 | US 2008/0015196 A1<br>EP 1734820 A2 | |
| WO 2011/018894 A1 | 17 Feb. 2011 | (Family: none) | |
| WO 2018/118736 A1 | 28 Jun. 2018 | US 2020/0095261 A1<br>EP 3558306 A1 | |
| WO 2019/216294 A1 | 14 Nov. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Rome IV (Functional Gastrointestinal Disorders. Rome Foundation, INC, 2016
- *Gastroenterology,* 2016, vol. 150 (6), 1257-1261
- *Advances in Clinical Experimental Medicine,* 2016, vol. 25 (1), 199-206
- *Pharmacolical Reviews,* 1998, vol. 50 (2), 279-290
- *British Journal of Pharmacology,* 2006, vol. 148 (5), 565-578
- *Trends in Pharmacological Sciences,* 2017, vol. 38 (9), 837-847
- *Nature,* 2012, vol. 482, 552-556
- *Acta gastroenterologica latinoamericana,* 1991, vol. 21 (1), 3-9